(19) Europäisches Patentamt
European Patent Office
Office européen des brevets

(11) EP 1 843 781 B1

(12) **EUROPEAN PATENT SPECIFICATION**

(45) Date of publication and mention of the grant of the patent:
**18.03.2015 Bulletin 2015/12**

(21) Application number: **06702098.2**

(22) Date of filing: **02.01.2006**

(51) Int Cl.:
***A61K 38/17*** *(2006.01)*
***A61K 38/46*** *(2006.01)*
***A61P 11/06*** *(2006.01)*
***G01N 33/564*** *(2006.01)*
***G01N 33/68*** *(2006.01)*

(86) International application number:
**PCT/KR2006/000005**

(87) International publication number:
**WO 2006/073254 (13.07.2006 Gazette 2006/28)**

(54) **COMPOSITION FOR PREVENTION, TREATMENT AND DIAGNOSIS OF CHRONIC INFLAMMATORY AIRWAY DISEASES**

ZUSAMMENSETZUNG ZUR PRÄVENTION, BEHANDLUNG UND DIAGNOSE VON CHRONISCHEN ENTZÜNDLICHEN ATEMWEGSERKRANKUNGEN

COMPOSITION POUR LA PREVENTION, LE TRAITEMENT ET LE DIAGNOSTIC DE MALADIES CHRONIQUES INFLAMMATOIRES DES VOIES AERIENNES

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.01.2005 KR 20050000098**
**18.02.2005 KR 20050013584**
**30.12.2005 KR 20050135454**

(43) Date of publication of application:
**17.10.2007 Bulletin 2007/42**

(73) Proprietors:
- **Jeon, Sook-yeong**
**Yongin-si,**
**Gyeonggi-do 449-843 (KR)**
- **Nahm, Dong-ho**
**Yongin-si,**
**Gyeonggi-do 449-843 (KR)**

(72) Inventors:
- **Jeon, Sook-yeong**
**Yongin-si,**
**Gyeonggi-do 449-843 (KR)**
- **Nahm, Dong-ho**
**Yongin-si,**
**Gyeonggi-do 449-843 (KR)**

(74) Representative: **Hoffmann Eitle**
**Patent- und Rechtsanwälte PartmbB**
**Arabellastraße 30**
**81925 München (DE)**

(56) References cited:
**WO-A2-2005/032572**
**US-A1- 2004 121 343**

- **ISHIGURO A. ET AL.: 'Identification of Candida albicans antigens reactive with immunoglobulin E antibody of human sera' INFECT. IMMUN. vol. 60, no. 4, 1992, pages 1550 - 1557, XP002116908**
- **WINKLER B. ET AL.: 'Mucosal tolerance as therapy of type I allergy: intranasal application of recombinant Bet v 1, the major birch pollen allergen, leads to the suppression of allergic immune responses and airway inflammation in sensitized mice' CLIN. EXP. ALLERGY vol. 32, no. 1, 2002, pages 30 - 36, XP008119936**
- **NITTNER-MARSZALSKA M. ET AL.: 'Skin prick test response to enzyme enolase of the baker's yeast in diagnosis of respiratory allergy' MED. SCI. MONIT. vol. 7, no. 1, 2001, pages 121 - 124, XP008120065**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The present invention relates to compositions for prevention, treatment, and diagnosis of chronic inflammatory airway diseases.

### BACKGROUND ART

The 'chronic inflammatory airway diseases' used in the present invention is defined as a comprehensive term including various diseases characterized by chronic airway inflammations and subsequent tissue damages involving airways including trachea, bronchus, bronchiole, and alveolus (Wardlaw A, et al. Clin Exp Allergy 2005:35:1254-62). Although bronchial asthma and chronic obstructive pulmonary disease (COPD) among the chronic inflammatory airway diseases are currently defined differently, there is a significant number of patients who have characteristic features of both bronchial asthma and COPD and it is difficult to exclusively classify to either of both diseases (Guerra S, Curr Opin Pulm Med 2005:11:7-13). Because there has been a "Dutch Hypothesis" that bronchial asthma and COPD are different phenotypes of one disease entity induced by common pathogenetic mechanism, chronic inflammatory airway diseases are intended to comprise both bronchial asthma and COPD in the present invention.

Bronchial asthma is a chronic inflammatory disease of the airways characterized by hyper-responsiveness of airways, episodic developments of airway narrowing, and difficult breathing and there are no methods to cure this disease completely yet (Global Initiative for Asthma. NIH Publication No. 02-3659. 2002). It has been estimated that 5 to 10 percent of patients with asthma have severe asthma that has not been effectively controlled by current drug treatment, and these patients have been exposed to high risk of asthma-related death (Global Initiative for Asthma. NIH Publication No. 02-3659, 2002).

Traditionally, an allergic immune response to common environmental agents such as house dust mites, pollens or the like (allergens) has been regarded as an important cause responsible for the development of airway inflammation, thereby asthma symptoms including cough, dyspnea and the like in patients with bronchial asthma (Lemanske, R.F., Jr et al., JAMA 1997; 278:1855-1873). However, allergic response to common environmental agents cannot be detected in a significant proportion (about 30% - 50%) of patients with bronchial asthma (Pearce, N. et al., Thorax 1999; 54:268-272), and the pathogenetic mechanism responsible for the development of airway inflammation in patients with non-allergic asthma is not determined yet (Global Initiative for Asthma. NIH Publication No. 02-3659. 2002, p50-66).

Airway epithelium has been suggested as a target for the inflammatory response in bronchial asthma on the basis of pathological studies (Montefort, S. et al., Clin Exp Allergy 1992: 22:511-520), and the severe asthma has been suggested to be an airway epithelial disease induced by abnormality of airway epithelial cells (Chanez P. Eur Respir J 2005;25:945-6). Thus, besides currently known allergic mechanism, the mechanism inducing a chronic airway inflammation, and especially the mechanism causing severe asthma have been suggested as major future research subjects (Global Initiative for Asthma. NIH Publication No. 02-3659, 2002, p50-66).

In a previous report, clinical features compatible with human bronchial asthma (acutely developed breathing difficulties and difficulties in expiration) were observed and documented by auscultation in normal rabbits after repeated intravenous administrations of serum samples of rats immunized by rabbit lung tissue and final administration of the immunized rat serum samples by inhalation route. However, administration of normal rat serum samples to normal rabbits in the same above manner did not result in significant changes. Thus, it was confirmed that antibodies to lung tissues could induce asthma. In that report, pathological examination of lung and airway tissues of rabbits received immunized rat serum samples showed constrictions of bronchioles (compatible with bronchial asthma) and emphysematous changes of lung tissues, and inflammations of vessel walls of small arteries in lung tissue (Karol SA, et al. Vrach Delo 1966;4:28-32). The deposition of IgG antibodies and complement in the bronchial mucosa of asthmatic patients who died of a fatal asthmatic attack has been reported (Callerame ML, et al., N Eng J Med 1971; 284: 459-64). Also, the analysis of bronchial tissue samples obtained by bronchoscopic biopsies from patients with adult-onset asthma showed the depositions of IgG antibodies and complement in the bronchial epithelial cells (Molina, C. et al., Clin Allergy 1977: 7: 137-45). On the basis of these reports, bronchial asthma has been recently suggested to be an autoimmune disease (Rottem M, Shoenfeld Y. Int Arch Allergy Immunol 2003;132:210-4).

However, a direct demonstration of a causal relationship between autoimmunity and asthma could not be established due to lack of identification of relevant autoantigen logically associated with chronic airway inflammation.

Although inventors have recently identified the cytokeratin 18 protein as an airway epithelial autoantigen associated with non-allergic asthma, the autoantibody test has not yet been used for the diagnosis or classification of bronchial asthma due to lack of identification of clinically more important autoantigens associated with severe asthma.

Especially, there is no simple laboratory test which can be used for the detection, classification, and diagnosis of specific bronchial asthma phenotypes such as severe asthma that is not effectively controlled by typical therapies and consequently resulted to have high risks of asthma-related death, or aspirin-hypersensitive asthma (asthma with aspirin-hypersensitivity) having risks of experiencing acute near fatal severe exacerbation of asthma in case of administration of aspirin or other nonsteroidal anti-inflammatory drugs.

WO 2005/032572 A2 concerns means and methods for the recruitment and identification of stem cells. It also discloses the use of alpha-erolase to recruit bone marrrow stem/progenitor cells as a medicament.

Effective methods or drugs for the prevention or treatment of bronchial asthma such as severe asthma or asprin-hypersensitive asthma that can fundamentally improve the disease on the basis of etiology and prevent asthma-related death have not been developed yet.

Chronic obstructive pulmonary disease (COPD) is currently defined as chronic disease state showing irreversible obstruction of airways resulting from the progression of two major underlying diseases including chronic bronchitis and emphysema. Chronic bronchitis is defined clinically as the persistence of cough, sputum, and difficult breathing, and emphysema is defined histopathologically as an irrevesible change of airway walls distal to the terminal bronchiole and clinically shows slowly progressive respiratory difficulties. COPD is currently the fourth leading cause of death in the United States and Europe, and causes of death in patients with COPD are complications of the disease such as respiratory failure or infection (GOLD workshop summary, Am J Respir Crit Care Med 2001; 163:1256-1276).

In some portion of patients with long-standing asthma, irreversible obstruction of airway which is difficult to distinguish with COPD can be developed, and thus bronchial asthma can be progressed to COPD (Celli BR, et al., Eur Respir J 2004; 23:932-46). Significant reversible improvement of airway obstruction after short-term or long-term pharmacological treatments with inhalations of bronchodilators and steroids has been documented in a significant number of patients meeting current diagnostic criteria for COPD (GOLD workshop summary. Am J Respir Crit Care Med 2001; 163:1256-1276). Therefore, there is a significant number of patients satisfying definition criteria for both bronchial asthma and COPD (Guerra S, Curr Opin Pulm Med 2005;11:17-13).

Currntly, smoking is regarded as the most important risk factor for the development of COPD. And secretions of many inflammatory mediators (IL-8 etc.) from airway epithelial cells stimulated by smoking and other factors including air pollution or chronic infection by bacteria or virus are considered to be responsible for the development of chronic inflammation of airway tissue in COPD. However, the reason for the chronic persistence of inflammation after acute inflammation induced by those external stimuli is not determined yet. Accordingly, it is suggested that other pathogenetic mechanisms besides smoking may be involved in the pathogenetic mechanism of COPD (O'Byrne PM. Postma DS. Am J Respir Crit Care Med 1999;159:S41-S66). Especially, the reasons for the persistence of airway inflammation and apoptosis of airway epithelial cells in COPD patients even after stop of smoking are not explained yet (Hodge S et al., Eur Respir J 2005;25:447-54). The etiology and pathogenetic mechanism of COPD are not determined yet, and therefore, fundamental treatment of COPD is difficult now. The COPD patients having advanced disease and severely decreased pulmonary function are at a high risk of the disease-associated premature death (GOLD workshop summary, Am J Respir Crit Care Med 2001;163:1256-1276).

Detection of autoantibodies to antigens of airway or lung tissues in COPD patients has been reported (Wagner V, et al., Acta Allergol 1965;20:1-9). Although an autoimmune hypothesis that COPD has been caused by autoimmune mechanism has been proposed on the basis of the previous study (Agusti A, et al., Thorax 2003;58:832-834), this hypothesis has not been clearly demonstrated because an autoantigen reacting with autoantibodies in the blood of patients with COPD has not been identified yet.

Recently, it has been demonstrated that pulmonary emphysema (one of major underlying diseases causing COPD) could have been developed in animals by autoimmune response against the vascular endothelial autoantigen (Taraseviciene-Stewart L, et al., Am J Respir Crit Care Med. 2005:171:734-42). In the above study, it has been reported that rats immunized by antigens extracted from human vascular endothelial cells to induce autoimmune response developed emphysema, and passive transfer of CD4+ cells from spleens of the above emphysema-developed rats to naive rats resulted in emphysema in recipient rats, and emphysema has been also caused in mouse after injection of anti-endothelial cell antibodies obtained from serum of rats immunized with vascular endothelial cells (Taraseviciene-Stewart L, et al., Am J Respir Crit Care Med. 2005:171:734-42). This study showed that antibodies to vascular endothelial cell antigens were sufficient to trigger the development of emphysema, although target autoantigens involved in development of the disease was not identified yet.

An alpha-enolase, which is identified as a target autoantigen of chronic inflammatory airway diseases in the present invention, is a key glycolytic enzyme and is mainly a cytosolic protein but is also expressed on the cell surface. This enzyme is ubiquitously expressed in the cytoplasm and cell surfaces of various cell types, including epithelial cells, endothelial cells, and hematopoietic cells and functions as a plasminogen receptor, and this indicates that alpha-enolase plays an important role in the fibrinolytic system (Pancholi V., Cell Mol Life Sci. 2001;58:902-920). Although alpha-enolase has been identified as a target autoantigen of various chronic inflammatory disease (Pancholi V., Cell Mol Life Sci. 2001;58:902-920), it has never been identified as a target autoantigen associated with chronic inflammatory airway diseases such as bronchial asthma or COPD.

Human alpha-enolase protein is composed of 434 amino acids, and the amino acid sequence is reported in the previous paper (Giallongo A, et al, Proc. Natl. Acad. Sci. U.S.A. 1986;83:6741-6745), and the degree of homology among the amino acid sequences of mammalian alpha-enolase proteins is reported to be very high (Pancholi V., Cell Mol Life Sci. 2001;58:902-920).

The present inventors judged that autoantigen proteins involved in the development of bronchial asthma and COPD might be present in the airway epithelial cells on the basis of previous reports and results from deductive ratiocination of present inventors, and therefore, analyzed the airway epithelial autoantigen proteins reacting with IgG autoantibodies by immunoblot method using proteins from cultured human airway epithelial cells.

Consequently, the inventors discovered autoantibodies to airway epithelial cells in serum samples of patients with bronchial asthma or COPD, and identified that the airway epithelial autoantigen was alpha-enolase protein. Also, inventors demonstrated significant inhibitions of autoantibody-induced cytotoxicity of airway epithelial cells and autoantibody-induced secretions of proinflammatory cytokines from airway epithelial cells by adsorption of autoantibodies from patients with bronchial asthma or COPD with alpha-enolase protein, and thereby the inventors made the present invention.

## DISCLOSURE OF INVENTION

It is an object of the present invention to provide a pharmaceutical composition for use in preventing or treating chronic inflammatory airway diseases comprising alpha-enolase protein as an active ingredient.

It is another object of the present invention to provide the use of a diagnostic composition for diagnosing chronic inflammatory airway diseases comprising alpha-enolase protein.

It is still another object of the present invention to provide a diagnostic composition for diagnosing chronic inflammatory airway diseases comprising alpha-enolase protein and cytokeratin 18 protein simultaneously.

It is yet another object of the present invention to provide the use of a composition for screening therapeutic agents for chronic inflammatory airway diseases comprising one or more of alpha-enolase protein, antibodies to alpha-enolase protein, and autoantibodies to alpha-enolase from patients with chronic inflammatory airway diseases.

The present invention provides a pharmaceutical composition for use in preventing or treating chronic inflammatory airway diseases comprising alpha-enolase protein as an active ingredient.

Alpha-enolase protein used in the present compositions for diagnosing or treating chronic inflammatory airway diseases, or screening therapeutinc agent for chronic inflammatory airway diseases, can be originated from mammals including human, mouse, rat, rabbit, cow, pig, and goat.

Alpha-enolase protein used in the present invention can be proteins having an amino acid sequence of SEQ ID NO: 1 or an amino acid sequence of SEQ ID NO: 2, or their polypeptide fragments retaining equal physiological activity to the alpha-enolase proteins.

In the present invention, the above polypeptide fragments mean polypeptides retaining minimal autoantigenic epitope binding with autoantibodies from patients with chronic inflammatory diseases.

The amino acid sequence of SEQ ID NO: 1 is an amino acid sequence of human (*Homo sapiens*) alpha-enolase protein (NCBI accession no. P06733), and an amino acid sequence of SEQ ID NO: 2 is an amino acid sequence of mouse (*Mus musculus*) alpha-enolase protein (NCBI accession no. P177182).

Also, the above alpha-enolase protein can be one of proteins expressed from the DNA base sequence of SEQ ID NO: 3 or the DNA base sequences with polymorphisms in the DNA base sequence of SEQ ID NO: 3.

The above SEQ ID NO: 3 is an mRNA sequence (NCBI accession no. M14628) including cDNA sequence (CDS) of human (*Homo sapiens*) alpha-enolase.

Also, alpha-enolase protein of the present invention can be obtained by isolating and purifying from cells or tissues of mammals, or microorganisms. Or, alpha-enolase protein can be produced by recombinant genetic engineering technology using either one of amino acid sequences of SEQ ID NO: 1 or SEQ ID NO: 2 or DNA base sequences encoding fragments of amino acid sequences of SEQ ID NO: 1 or SEQ ID NO: 2 or DNA base sequences of SEQ ID NO: 3 or DNA base sequences with polymorphisms in the DNA base sequence of SEQ ID NO: 3.

The chronic inflammatory airway diseases' used in the present invention is defined as comprehensive term including various diseases characterized by chronic airway inflammations and subsequent damages of airway tissues involving trachea, bronchus, bronchiole and alveolus. In the present invention, the above chronic inflammatory airway diseases especially include bronchial asthma and chronic obstructive pulmonary disease. Also, in the present invention, the above bronchial asthma includes mild asthma, moderate asthma, severe asthma and aspirin-hypersensitive asthma.

The present invention is based on the fact identifying alpha-enolase protein as an airway epithelial autoantigen binding with IgG autoantibodies in the blood samples of patients with bronchial asthma, especially patients with severe athma, and patients with chronic obstructive pulmonary disease, for the first time.

It can be judged from the previous reports and results of Examples in the present invention that autoantibodies in the blood of patients with bronchial asthma or chronic obstructive pulmonary disease can directly induce cytotoxicity to airway epithelial cells through binding to alpha-enolase proteins of airway epithelial cells or indirectly induce cytotoxicity to airway epithelial cells and chronic inflammation of airway tissue through the formation of immune complexes consisted of autoantibodies and autoantigen and secondary complement activation and chemotaxis of inflammatory cells. And this chronic airway inflammation can cause clinical symptoms of bronchial asthma and chronic obstructive pulmonary disease through the developments of airway constriction, airway hyperresponsiveness, and irreversible structural changes of airway tissues.

In the Examples of the present invention, IgG antibodies purified from blood samples of patients with bronchial asthma and chronic obstructive pulmonary disease induced cytotoxicity to airway epithelial cells and secretion of proinflammatory cytokines. Also, the administration of alpha-enolase protein of the present invention into the model of IgG antibody-induced airway epithelial cell cytotoxicity, induced adsorptions of autoantibodies in the blood of patients with bronchial asthma and chronic obstructive pulmonary disease, and thereby inhibited autoantibody-induced cytotoxicity to airway epithelial cells and autoantibody-induced secretion of proinflammatory cytokines from airway epithelial cells.

Thus, the pharmaceutical compositon comprising alpha-enolase protein according to the present invention can be used as a medicament preventing, alleviating or treating chronic inflammatory airway diseases including bronchial asthma and chronic obstructive pulmonary disease.

The pharmaceutical compositon comprising alpha-enolase protein as an active ingredient may further comprise pharmaceutically and physiologically acceptable additives besides the active ingredient. Such additives may include, for example, excipients, disintergrating agents, sweeting agents, binding agents, coating agents, inflating agents, lubricants, glidants, flavoring agents, solubilizers, etc.

The pharmaceutical compositon comprising alpha-enolase protein as an active ingredient may further comprise one or more pharmaceutically acceptable carriers to be formulated appropriately for administration.

For liquid formulation, the pharmaceutically acceptable carriers should be sterilized and suitable to living bodies. For example, the pharmaceutically acceptable carriers may include saline, sterilized water, linger solution, bufferd saline, albumin injection solution, dextrose solution, malto dextrine solution, glycerin, ethanol, or the mixture of one or more of the above ingredients. If necessary, other common additives can be added, such as antioxidants, buffers, bacteriostatic agents, etc. Also, diluting agents, dispersing agents, surfactants, binders or lubricants can be further added in order to formulate the composition to injection formulations such as aquous solution, suspension, emulsion, pills, capsules, granules, Tablets, etc. Furtherrmore, the present pharmaceutical compositon can be appropriately formulated depending on each disease or ingredient of the composition, by using the disclosed method in Remington's Pharmaceutical Science, Mack Publishing Company, Easton PA, as a preferable method in the art.

Formulation type of the present pharmaceutical compositon comprising alpha-enolase protein as an active ingredient, can be granules, powder, coated tablets, tablets, capsules, suppositories, syrup, juice, suspensions, emulsions, drops, injectable liquid formulation or slowly-released formulation of active compound, etc.

The present pharmaceutical compositon comprising alpha-enolase protein as an active ingredient, can be administered intravenously, intraarterially, intraperitoneally, intramuscularly, intrasternally, percutaneously, intranasally, rectally, orally, intraocularly, intradermally, locally, or by inhalation, according to ordinary methods.

The dosage of the present pharmaceutical composition comprising alpha-enolase protein as an active ingredient means an effectective amount to inhibit or treat damage or inflammatory reaction of airway epithelial cells and tissues. Thus, this daosage can be modified depending on various factors such as the kind of a disease to be treated, severity of the disease, kinds and amounts of active ingredients and other ingredients contained in the composition, age, weight, general health status and sex of the patient, diet, time and route of administration, secretion rate of the composition, period of treatment, drugs used simultaneously, etc. In adults, when alpha-enolase proteins are administrated one or several times per day, the preferable dosage of alpha-enolase proteins is 0.01 mg/kg ~ 100 mg/kg.

Also, the present invention provides a pharmaceutical composition for use in preventing or treating chronic inflammatory airway diseases by administering alpha-enolase protein. Preferably, the above chronic inflammatory airway diseases can be bronchial asthma including severe asthma and aspirin-hypersensitive asthma, or chronic obstructive pulmonary disease.

The present invention shows that the administration of alpha-enolase proteins can adsorb autoantibodies in the blood of patients with chronic inflammatory airway diseases thereby inhibiting autoantibody-induced cytotoxicity to airway epithelial cells and inhibiting autoantibody-induced secretion of proinflammatory cytokines from airway epithelial cells.

The present invention provides a pharmaceutical composition for use in preventing or treating chronic inflammatory airway diseases by administering alpha-enolase protein. In this method, alpha-enolase protein can be administrated intravenously, intraarterially, intraperitoneally, intramuscular, intrastemally, percutaneously, intranasally, rectally, orally, intraocularly, intradermally, locally, or by inhalation, according to ordinary methods.

In the use in preventing or treating chronic inflammatory airway diseases by administering alpha-enolase protein, the dosage of alpha-enolase proteins to be administrated can be referred as an effective amount to inhibit or treat autoantibody-induced cytotoxicity to airway epithelial cells or autoantibody-induced secretion of proinflammatory cytokine from airway epithelial cells, or secondary inflammatory reaction of airway tissues caused by the formation of immune complex consisted of autoantigen-autoantibodies. In adults, when alpha-enolase proteins are administrated one or several times per day, the preferable dosage of alpha-enolase proteins is 0.01 mg/kg ~ 100 mg/kg. This dosage can be modified depending on various factors such as the kind of a disease to be treated, severity of the disease, kinds and amounts of active ingredients and other ingredients contained in the composition, age, weight, general health status and sex of the patient, diet, time or route of administration, secretion rate of composition, period of treatment, drugs used simultaneously, etc.

Also, the present invention provides the use of alpha-enolase protein for the manufacture of a medicament for chronic inflammatory airway diseases such as bronchial asthma or chronic obstructive pulmonary disease.

Further, the present invention provides the use of a diagnostic composition comprising alpha-enolase protein, for diagnosing chronic inflammatory airway diseases such as bronchial asthma or chronic obstructive pulmonary disease.

The alpha-enolase protein contained in the present diagnostic composition can be proteins having the amino acid sequence of SEQ ID NO: 1 or the amino acid sequence of SEQ ID NO: 2, or their polypeptide fragments retaining equal physiological activity to the alpha-enolase proteins, and may be originated from mammals including human, rat, mouse, rabbit, cow, pig, goat, etc.

The diagnostic composition comprising alpha-enolase protein for use according to the present invention can react with biological samples of patients with chronic inflammatory airway diseases such as bronchial asthma or chronic obstructive pulmonary disease, thereby showing positive results. Thus, the diagnostic composition can be used for the diagnosis of chronic inflammatory airway diseases such as bronchial asthma or chronic obstructive pulmonary disease.

From the prior reports of immunological studies for other autoimmune dieases, it was known that in case of patients showing IgG autoantibodies to specific autoantigen proteins in blood, a considerable number of patients has antigen-specific T-cell reaction to the same autoantigen protein, and it is useful to test delayed type hypersensitivity after intradermal injection of the same autoantigen proteins reacting with IgG autoantibodies in the patients (Beales PE et al, Autoimmunity 2000;32:109-113). In prior arts, skin tests of tuberculin type have been used, in which tests are performed by injecting specific antigen to subjects, intradermally, and testing the injected skin part after 24 to 72 hours of the injection to measuring the size of skin swelling (induration), thus determining the presence of delayed-type hypersensitivity reaction or late-onset skin reaction to the above antigen.

Accordingly, the diagnostic composition comprising alpha-enolase protein can be applied to the use of the diagnostic composition to detecting patients showing autoimmune response to alpha-enolase protein among patients with chronic inflammatory airway diseases including bronchial asthma or chronic obstructive pulmonary diseases by measuring the size of skin swelling after 24 to 72 hours of the intradermal injection of alpha-enolase protein.

Also, the present invention provides a diagnostic composition containing alpha-enolase protein for use in a method of diagnosing chronic inflammatory airway diseases including bronchial asthma or chronic obstructive pulmonary diseases by using a diagnostic composition comprising alpha-enolase protein wherein the said method of diagnosing chronic inflammatory airway diseases comprises the following steps:

a) selecting a subject suspected to have chronic inflammatory airway diseases;
b) injecting intradermally any one of the diagnostic compositions of claims 13 to 18 to the subject; and
c) diagnosing the chronic inflammatory airway diseases by examining the injected skin part after 24 to 72 hours of the injection and detecting the presence of the skin swelling and measuring the size of skin swelling, thus determining the presence of delayed-type hypersensitivity reaction or late skin reaction to alpha-enolase protein.

Also, the present inventon provides a diagnostic composition comprising alpha-enolase protein and cytokeratin 18 protein simultaneously.

The present invention also provides a diagnostic composition for diagnosing chronic inflammatory airway diseases comprising alpha-enolase protein and cytokeratin 18 protein simultaneously.

Alpha-enolase proteins used in this diagnostic composition are the same as those used for the above diagnostic composition. And cytokeratin 18 protein can be proteins having the amino acid sequence of SEQ ID NO: 4 or their polypeptide fragment retaining equal physiological activity to cytokeratin 18 proteins. The amino acid sequence of SEQ ID NO: 4 is the amino acid sequence of human (*Homo sapiens*) cytokeratin 18 protein (NCBI accession no. P05783).

Cytokeratin 18 protein can be originated from mammals including human, mouse, rat, rabbit, pig, cow, goat, etc. The present inventors' prior report disclosed that bovine (cow) cytokeratin 18 protein showed similar antigenicity to human cytokeratin 18 protein, and reacted with IgG antibodies in the serum samples of patients with bronchial asthma (Nahm DH, e tal. Am J Respir Crit Care Med 2002;165:1536-9).

Also, cytokeratin 18 protein can be one or more proteins expressed from one of DNA base sequence of SEQ ID NO: 5 or DNA base sequences with polymorphism in SEQ ID NO: 5.

SEQ ID NO: 5 is mRNA sequence comprising cDNA sequence (CDS) of cytokeratin 18 protein in human *Homo sapiens* (NCBI accession no. NM_199187)

Also, cytokeratin 18 protein can be obtained by isolating them from cells or tissues of mammals, or microorganisms and purifying them. Or, cytokeratin 18 protein can be obtained by recombinant genetic engineering technology using one of amino acid sequence of SEQ ID NO: 4, DNA sequence encoding their fragment, DNA base sequence of SEQ ID NO: 5 or DNA base sequences with polymorphism in SEQ ID NO: 5, therby expressing them in cells or tissues of mammals, or microorganisms and purifying them.

The present diagnostic composition comprising alpha-enolase protein and cytokeratin 18 protein simultaneously can be used for the detection, diagnosis, and classification of chronic inflammatory airway diseases such as severe asthma, aspirin-hypersensitive asthma, and COPD with higher diagnostic sensitivity as shown in the present Examples.

The diagnostic composition can comprise buffer or reaction solution which makes maintain physiological acivity or structure of proteins, besides the proteins. Also, the diagnostic composition can be provided in the form of powder, or in the solubilized state in an appropriate buffer, or maintained at 4 °C. in order to maintain stability.

A diagnostic kit for the diagnosing chronic inflammatory airway diseases comprising diagnostic composition comprising alpha-enolase protein is described.

Also, a diagnostic kit for diagnosing chronic inflammatory airway diseases comprising diagnostic composition comprising alpha-enolase protein and cytokeratin 18 protein simultaneously for diagnosing chronic inflammatory airway diseases is described.

The diagnostic kit can comprise buffer or reaction solution which makes to maintain physiological acivity or structure of proteins, besides alpha-enolase protein or cytokeratin 18 protein. Further, the proteins can be provided in the form of powder, or in the solubilized state in an appropriate buffer, or maintained at 4 °C, in order to maintain stability.

The diagnostic kit can comprise components other than alpha-enolase protein or cytokeratin 18 protein. For the positive control result, the diagnostic kit can comprise polyclonal antibodies or monoclonal antibodies to alpha-enolase protein or cytokeratin 18 protein from mammals, or human's biological samples, etc. of which autoantibodies to alpha-enolase protein or cytokeratin 18 protein are positive. For the negative control result, the diagnostic kit can comprise other antibodies or buffers. If necessary, the diagnostic kit can further comprise other components required for the detection of autoantibodies to alpha-enolase protein or cytokeratin 18 protein from biological samples of subjects.

Other components required for immunodetecting reaction include anti-human IgG antibodies produced from rat, mouse, rabbit, cow, pig, or goat, etc., as the secondary antibodies, and colorizing agents, buffers, etc.

As the secondary antibodies, alkaline phosphatase (AP)-conjugated, horse radish peroxidase (HRP)-conjugated, biotin-conjugated antibodies, or fluorescent material(for example, rhodamine, Texas Red, fluorescein, phycoerythrin, etc.)-conjugated antibodies can be used.

The biotin-conjugated secondary antibodies can use AP or HRF enzyme-conjugated avidin, AP-conjugated secondary antibodies can use BCIP/NBT as color couplers, and HPR-conjugated secondary antibodies can use DAB (diaminobenzidine), etc. as color couplers, thereby inducing color reaction to determine the presence of autoantibodies. The secondary antibodies to which fluorescent materials are conjugated, can be monitored on a fluorescence microscope. Thus, from the fluorescence microscopy, the presence of autoantibodies can be determined.

The assay methods applied to the kit are known to those skilled in the art. The assay methods to be used for the kit include any techniques that can detect antigen-antibody reaction, such as fluorescence immunoassays, enzyme-substrate color reaction, enzyme immunoassays, immunoblotting, immunoprecipitation assay, antigen-antibody agglutination immunoassays, light-scattering immunoassays, radioimmunoassay, flow cytometry, complement-fixing method, etc.

The kit can further comprise tubes, wells or plates required for mixing each component, or covering letter describing the way of using, if necessary.

The present invention describes a method for diagnosing chronic inflammatory airway diseases such as bronchial asthma or chronic obstructive pulmonary disease by using diagnostic compositions comprising alpha-enolase protein as an active ingredient.

The method comprises the following steps:

(a) obtaining biological samples of a subject suspected to have chronic inflammatory airway diseases such as bronchial asthma or chronic obstructive pulmonary disease;
(b) contacting the biological samples with alpha-enolase protein, thereby inducing the formation of immune complex; and
(c) diagnosing the chronic inflammatory airway diseases in the subject by detecting the immune complex, thereby determining the presence of autoantibodies to alpha-enolase protein.

The present invention describes a method for diagnosing chronic inflammatory airway diseases such as bronchial asthma or chronic obstructive pulmonary disease by using diagnostic compositions comprising alpha-enolase protein and cytokeratin 18 protein as active ingredients.

The method comprises the following steps:

(a) obtaining biological samples of a subject suspected to have chronic inflammatory airway diseases such as bronchial asthma or chronic obstructive pulmonary diseases:

(b) contacting the biological samples with alpha-enolase protein and cytokeratin 18 protein, thereby inducing the formation of immune complex; and

(c) diagnosing the chronic inflammatory airway diseases in the subject by detecting the immune complex, thereby determining the presence of autoantibodies to alpha-enolase protein and cytokeratin 18 proteins.

In step (a) of the present diagnosing method, the biological samples can include any fluid which can be isolated and collected from human bodies, such as blood, plasma, serum, urine, tears, salivar, sputum, nasal secretion, bronchial secretion, bronchial washing fluid, pulmonary secretion, alveolus washing fluid, etc.

In step (b) of the present diagnosing method, the formation of immune complex can use immunoblotting, ELISA, etc., and can use any methods for detecting the antigen-antibody reaction, which are ordinary in the art.

Step (c) of the present diagnosing method is a step determining the formation of immune complex by inducing color reactions or detecting fluorescence of immune complex, and it can use any methods for detecting antigen-antibody reaction, which are ordinary in the art. If the presence of immune complex is confirmed, this result means that the subject has chronic inflammatory airway diseases such as bronchial asthma or chronic obstructive pulmonary diseases. For the positive control result, instead of the subject's biological samples, what can be used are polyclonal antibodies or monoclonal antibodies to one or more of alpha-enolase protein or cytokeratin 18 protein from mammals, or human's biological samples, etc. of which autoantibodies to one or more of alpha-enolase protein or cytokeratin 18 protein are positive. For the negative control result, what can be used are antibodies or buffers other than the antibodies to one or more of alpha-enolase protein or cytokeratin 18 protein.

Also, the present invention describes a method of diagnosing chronic inflammatory airway diseases including bronchial asthma and chronic obstructive pulmonary disease by injecting the diagnostic composition comprising alpha-enolase protein intradermally to the subjects who are suspected to have chronic inflammatory airway diseases, thereby determining the presence of autoimmune response to alpha-enolase protein by the measuring the degree of delayed-type hypersensitivity reaction to the above diagnostic composition.

The diagnosing method comprises the following steps:

a) selecting subjects who are suspected to have chronic inflammatory airway diseases;
b) injecting intradermally the diagnostic composition comprising alpha-enolase protein to the subjects; and
c) diagnosing chronic inflammatory airway diseases by examineing the injected skin part after 24 to 72 hours of the injection and detecting the presence of skin swelling and measuring the size of skin swelling, thereby determining the presence of delayed-type hypersensitivity reaction or late skin reaction to alpha-enolase protein.

By using the present diagnostic composition and diagnosing method, the result for the presence of bronchial asthma or chronic obstructive pulmonary diseases can be obtained easily and correctly determined. Therefore, it can be used effectively for clinical study in a large scale. Also, it can be used to screen therapeutic agents or to develope treatment methods for bronchial asthma or chronic obstructive pulmonary disesase.

Further, the present invention provides a composition and a method for detecting autoantibodies comprising alpha-enolase protein and cytokeratin 18 protein simultaneously.

The present invention provides a composition for detecting autoantibodies comprising alpha-enolase protein and cytokeratin 18 protein simultaneously.

The alpha-enolase protein contained in the present composition for diagnosing autoantibodies are proteins having the amino acid sequence of SEQ ID NO: 1 or the amino acid sequence of SEQ ID NO: 2, or their polypeptide fragments showing equal physiological activity to the above alpha-enolase proteins. And cytokeratin 18 proteins can be proteins having the amino acid sequence of SEQ ID NO: 4 or their polypeptide fragment showing equal physiological activity to the above cytokeratin 18 protein.

The alpha-enolase protein and cytokeratin 18 protein may be originated from mammals including human, mouse, rat, rabbit, pig, cow, goat, etc.

Also, the cytokeratin 18 proteins can be one or more proteins selected from proteins expressed from DNA base sequences of SEQ ID NO: 5 or DNA base sequences with polymorphism in SEQ ID NO: 5.

Also, cytokeratin 18 protein can be obtained by isolating and purifying from cells or tissues of mammals, or microorganism. Or, cytokeratin 18 protein can be produced by recombinant genetic engineering technology using one of DNA base sequence encoding the amino acid sequence of SEQ ID NO: 4 or their fragment, DNA base sequence of SEQ ID NO: 5 or DNA base sequence with polymorphism in SEQ ID NO: 4, therby isolating them from cells or tissues of mammals, or microorganisms and purifying them.

Such diagnostic composition for detecting autoantibodies comprising alpha-enolase protein and cytokeratin 18 protein simultaneously can be used for detecting autoantibodies from subjects including patients with chronic inflammatory airway diseases, with higher diagnosability, as shown in the present Examples.

The diagnostic composition for detecting autoantibodies can comprise buffers or reaction solutions which makes maintain physiological activity or structure of proteins, besides proteins. Also, the proteins can be provided in the form of powder, or solubilized state in an appropriate buffer, or maintained at 4°C, in order to maintain stability.

Also, a kit for detecting autoantibodies, comprising alpha-enolase protein and cytokeratin 18 protein simultaneously is described.

The kit for detecting autoantibodies can comprise buffers or reaction solutions which makes maintain physiological acivity or structure of proteins, besides alpha-enolase protein and cytokeratin 18 protein. Also, they can be provided in the form of powder or in the solubilized state in an appropriate buffer, or maintained at 4 °C, in order to maintain stability.

The kit for detecting autoantibodies can comprise components other than the alpha-enolase protein or cytokeratin 18 protein. For the positive control result, the kit can comprise polyclonal antibodies or monoclonal antibodies to alpha-enolase protein or cytokeratin 18 protein from mammals, or human's biological samples etc. of which autoantibodies to alpha-enolase protein or cytokeratin 18 protein are positive. For the negative control result, the kit can comprise other antibodies or buffers. If necessary, the kit can further comprise other components required for detecting autoantibodies to alpha-enolase protein or cytokeratin 18 proteins from biological samples of subjects.

Other components required for immunodetecting reation can be, for example, anti-human IgG antibodies produced from rat, mouse, rabbit, cow, pig, goat, etc., as the secondary antibodies, and colorizing agents, buffers, etc.

As the secondary antibodies, alkaline phosphatase (AP)-conjugated, horse radish peroxidase (HRP)-conjugated, biotin-conjugated antibodies, or fluorescent material (for example, rhodamine, Texas Red. fluorescein, phycoerythrin, etc.)-conjugated antibodies can be used.

The biotin-conjugated secondary antibodies can use AP or HRF enzyme-conjugated avidin, AP-conjugated secondary antibodies can use BCIP/NBT as color couplers, and HPR-conjugated secondary antibodies can use DAB (diaminobenzidine), etc. as color couplers, thereby inducing color reaction to determine the presence of autoantibodies. The secondary antibodies to which fluorescent materials are conjugated can be monitored on a fluorescence microscope. Through fluorescence microscopy, the presence of autoantibodies can be determined.

The assay methods applied to the kit are known to those skilled in the art. The assay methods used in the kit, can include any techniques that can detect antigen-antibody reaction, such as fluorescence immunoassays, enzyme-substrate color reaction, eyzyme immunoassays, immunoblotting, immunoprecipitation assay, antigens-antibodies agglutination immunoassays, light-scattering immunoassays, radioimmunoassay, flow cytometry, complement-fixing method, etc.

The kit can further comprise tubes, wells or plates required for mixing the components, or covering letter describing the way of using, if necessary.

The present invention provides a method of detecting autoantibodies by using compositions for detecting autoantibodies, wherein the compositions comprise alpha-enolase protein and cytokeratin 18 protein as active ingredients.

The method comprises the following steps:

(a) obtaining biological samples of a subject;
(b) contacting the biological samples with alpha-enolase protein and cytokeratin 18 protein, thereby inducing the formation of immune complex; and
(c) detecting the formation of the immune complex, thereby determining the presence of autoantibodies to alpha-enolase protein and cytokeratin 18 protein.

In step (a) of the present method for detecting autoantibodies, the biological samples can include any fluid which can be isolated and collected from human bodies such as blood, plasma, serum, urine, tears, salivar, sputum, nasal secretion, bronchial secretion, bronchial washing fluid, pulmonary secretion or alveolus washing fluid, etc.

In step (b) of the present method for detecting autoantibodies, the formation of the immune complex can use immunoblotting, ELISA, etc., and can use any methods for detecting antigen-antibody reaction, which are ordinary in the art.

Step (c) of the present method for detecting autoantibodies is a step detecting formation of the immune complex by inducing color reaction or detecting fluorescence of immune complex, and it can use any methods for detecting antigen-antibody reaction, which are ordinary in the art. If the presence of the immune complex is confirmed, this result means that the subject has autoantibodies to any one of alpha-enolase protein or cytokeratin 18 protein. For the positive control result, instead of subjects' biological samples, what can be used are polyclonal antibodies or monoclonal antibodies to one or more proteins among alpha-enolase protein or cytokeratin 18 protein from mammals, or human's biological samples, etc. of which antibodies to one or more of alpha-enolase protein or cytokeratin 18 protein are positive.

For the negative control result, what can be used are buffers or other antibodies besides antibodies to one or more proteins of alpha-enolase protein or cytokeratin 18 protein.

By using the composition, and the method for detecting autoantibodies according to the present invention, the result for the presence of autoantibodies to any one of alpha-enolase protein or cytokeratin 18 protein in the tested subject including patients with chronic inflammatory diseases can be obtained easily and correctly. Therefore, they can be used effectively for clinical study in a large scale. Also, they can be used for studying autoantibody-associated diseases.

A composition for screening a therapeutic agent for chronic inflammatory airway diseases, comprising one or more materials of alpha-enolase protein, antibodies to alpha-enolase protein, or autoantibodies to alpha-enolase from the patients with chronic inflammatory airway diseases, and a method for screening a therapeutic agent using this composition is described,

Alpha-enolase protein contained in the present composition for screening therapeutic agents can be poteins having the amino acid sequence of SEQ ID NO: I or the amino acid sequence of SEQ ID NO: 2, or proteins expressed from DNA base sequences of SEQ ID NO: 3 or DNA base sequences with polymorphisms in SEQ ID NO: 3, or polypeptide fragments of alpha-enolase protein showing equal physiological activity to alpha-enolase protein.

The antibodies to alpha-enolase protein contained in the present composition for screening therapeutic agents can be prepared for the alpha-enolase proteins, but it can be also obtained commonly or commercially in the art.

The compositions for screening a therapeutic agent can comprise buffer or reaction solution which makes maintain physiological acivity or structure of proteins, besides alpha-enolase protein, antibodies to alpha-enolase protein, or autoantibodies to alpha-enolase from the patients with chronic inflammatory airway diseases. Also, the composition can be provided in the form of powder, or in the solubilized state in an appropriate buffer, or maintained at 4°C, in order to maintain stability.

The present compositions for screening a therapeutic agent enable the selection of a therapeutic agent for chronic inflammatory airway diseases such as bronchial asthma of chronic obstructive pulmonary disease, by screening the material which inhibits binding between alpha-enolase proteins and autoantibodies to alpha-enolase from biological samples obtained from the patients with severe asthma or chronic obstructive pulmonary diseases, or inhibits cytotoxic reaction of the autoantibodies to alpha-enolase protein-expressing cells or inhibits secretion of inflammatory mediators including proinflammatory cytokine from alpha-enolase protein-expressing cells caused by the autoantibodies.

Also, the present invention provides a method of screening a therapeutic agent for chronic inflammatory airway diseases, by using compositions comprising one or more of alpha-enolase protein, antibodies to alpha-enolase protein, or autoantibodies to alpha-enolase from the patients with chronic inflammatory airway diseases, as target materials.

The method of screening a therapeutic agent for chronic inflammatory airway diseases comprises the following step:

(a) isolating autoantibodies from biological samples obtained from the patients with chronic inflammatory airway diseases such as bronchial asthma or chronic obstructive pulmonary diseases;
(b) contacting test materials with the autoantibodies obtained in step (a); and
(c) selecting a therapeutic agent for chronic inflammatory airway diseases by determining whether the test materials can inhibit binding of the autoantibodies to alpha-enolase protein, or inhibit cytotoxic effects of the autoantibodies to alpha-enolase protein-expressing cells or inhibit secretion of inflammatory mediators including proinflammatory cytokine from alpha-enolase protein-expressing cells caused by the autoantibodies.

In the present method of screening a therapeutic agent, the test materials can be nucleic acids, proteins, extracts, or compounds which are expected to have possibilities of inhibitor for chronic inflammatory airway diseases according to ordinary selecting method, or randomly selected.

In the present method for screening a therapeutic agent, for the confirmation of the reaction between the composition for screening and the test materials, common methods used to confirm protein-protein reaction including antigen-antibody reaction or protein-compound reaction can be used.

For example, the following methods can be used: a method which reacts alpha-enolase proteins or autoantibodies with the test material, thereby determining the activity, yeast two-hybrid method, screening method of phage-displayed peptide clone binding to alpha-enolase proteins, high throughput screening (HTS) method using natural product, chemical library etc., drug hit HTS method, cell-based screening method, or screening method using DNA array, etc.

In present inventon, matters relating to genetic engineering technology will be more clear from the contents of Sambrook's literature etc. (Sambrook, et al., Molecular Cloning, A Laboratory Manual, Cold Spring Harbor laboratory Press, Cold Spring Harbor, N. Y. (2001)).

## BRIEF DESCRIPTION OF THE DRAWINGS

Fig. 1 shows immunoblot analysis of human airway epithelial cell (A549) proteins reacting with IgG autoantibodies in serum samples of healthy controls and patients with severe asthma;
Fig. 2 shows the airway epithelial cell (A549) proteins separated by SDS-PAGE, and stained by coomassie blue;
Fig. 3 shows two-dimensional immunoblot analysis of human airway epithelial cell (A549) proteins reacting with IgG autoantibodies in a serum sample of a patient with severe asthma;
Fig. 4 shows two-dimensional immunoblot localization of alpha-enolase proteins in airway epithelial cell (A549) proteins using goat specific antibody to human alpha-enolase;
Fig. 5 shows immunoblot detection of IgG autoantibodies to recombinant human alpha-enolase proteins in serum samples of healthy controls and patients with severe asthma;
Fig. 6 shows immunoblot analysis of human airway epithelial cell (A549) proteins reacting with IgG autoantibodies in serum samples of healthy controls, emphysema patients having normal pulmonary function, and patients with COPD;
Fig. 7 shows immunoblot detection of IgG autoantibodies to recombinant human alpha-enolase protein in serum samples of healthy controls, emphysema patients with normal pulmonary function, and patients with COPD;
Fig. 8 shows detection of immune reaction bewteen purified recombinant human alpha-enolase proteins and IgG autoantibodies in serum samples of healthy controls, patients with severe asthma, and patients with COPD, by enzyme-linked immunosorbent assay (ELISA);
Fig. 9 shows immunoblot analysis of human airway epithelial cell (A549) proteins and mouse hepatoma cell (Hepa 1-6) proteins reacting with IgG autoantibodies in serum samples of two patients with severe asthma:
Fig. 10a shows the detection of antibody-induced cytotoxicity to human airway epithelial cells (A549) by IgG antibodies purified from patients with severe asthma, Fig. 10b shows the detection of antibody-induced cytotoxicity to human airway epithelial cells (A549) by IgG antibodies purified from patients with COPD, and Fig. 10c shows the detection of antibody-induced cytotoxicity to human airway epithelial cells (A549) by goat specific IgG antibodies to alpha-enolase protein or normal goat 1gG antibodies;
Fig. 11 shows inhibitions of IgG autoantibody-induced cytotoxicity to airway epithelial cells (A549) when the IgG antibodies from patients with severe asthma and patients with COPD were adsorbed with recombinant human alpha-enolase protein prior to addition to airway epithelial cells;
Fig. 12a shows the secretions of interleukin-8 (IL-8) from airway epithelial cells (A549) by addition of IgG antibodies from healthy controls and a patient with bronchial asthma; and
Fig. 12b shows the inhibition of the IgG antibody-induced secretion of interleukin-8 (IL-8) from airway epithelial cells (A549) when the IgG antibodies from a patient with bronchial asthma were adsorbed with recombinant human alpha-enolase protein prior to addition to airway epithelial cells.

## MODE FOR THE INVENTION

Hereinafter, the present invention is more specifically explained by the Examples. However, the following examples are provided for the purpose of illustration and are not intended to limit the scope of the present invention.

### [EXAMPLE 1] Identification of alpha-enolase as autoantigen in patients with bronchial asthma

(1-1) Subjects

The present inventors examined serum samples obtained from 78 patients with bronchial asthma (severe asthma) and 58 healthy controls.

All patients with bronchial asthma had typical clinical histories compatible with bronchial asthma, and showed a decrease in forced expiratory volume in one second ($FEV_1$) greater than 20% of baseline value following the inhalation of less than 8 mg methacholine/milliliter, or an increase in $FEV_1$ greater than 15% of the baseline measurement after the inhalation of a bronchodilator. All patients with bronchial asthma underwent skin-prick tests with 50 common aeroallergens (Bencard Co., Brentford, UK) and atopy was defined when mean wheal diameter of any one allergen was greater than 3 mm.

Severe asthma was defined as the case when patients had experienced at least one severe asthmatic exacerbation requiring for an emergency room visit or admission and intravenous administration of corticosteroid in the last year despite continuous standard therapies according to the guidelines by global initiative for asthma (Global Initiative for Asthma. NIH Publication No. 02-3659. 2002).

Also, aspirin-hypersensitivity was determined by a typical clinical history that asthma patients have been suffered from acute exacerbation of bronchial asthma after the administration of aspirin or other analgesic anti-inflammatory agent, or have been documented for the development of a significant acute asthmatic response after the inhalation of neublized lysine-aspirin according to prior reported method (Park HS. Clin Exp Allergy 1995; 25:28-40).

All serum samples of the subjects were stored at -20 °C.

(1-2) Cell Culture

The human airway epithelial cell line A549 (ATCC CCL-185; Giard et al, J Natl Cancer Inst 1973: 51:1417-23) was obtained from American Type Culture Collection (ATTC; VA, USA) and was cultured as recommend by ATCC.

(1-3) Extraction of cell protains and immunoblot analysis

Immunoblot analysis was performed with human airway epithelial cells and the serum samples obtained from healthy controls and patients with bronchial asthma according to the above Example (1-1), to identify human airway epithelial cell proteins which bind to IgG autoantibodies in the serum.

Firstly, in order to extract proteins from the human airway epithelial cell A549 in the above (1-2), the cultured cells were lysed in lysis buffer containing 10 mM Tris/HCl, pH 7.2, 2% sodium dodecyl sulphate (SDS), 158 mM NaCl, and 10 mM dithiothreitol.

The proteins in cell lysates were separated by discontinuous Sodium Dodecyl Sulfate-PolyacrylAmide Gel Electrophoresis (SDS-PAGE). Following electrophoresis, proteins were transferred onto a polyvinylidine difluoride (PVDF) membrane (Bio-Rad Laboratories. Hercules, CA). After the transfer, the PVDF membrane was treated with Tris-buffered saline (TBS) containing 5% bovine serum, 10% powdered skim milk and 0.1% Tween 20 for one or more hours, to block non-specific bindings of proteins to PVDF, and then the PVDF membrane was cut in 4mm strips. The cut strips were reacted with patients' serum diluted in the same buffer in 1:100 (v/v) for 2 hours at room temperature. After washing, the membrane was incubated with alkaline phosphates-conjugated goat anti-human IgG antibodies (Sigma Chemical Co., St. Louis, MO) for 2 hours at room temperature. After the final washing, the membrane was stained with a BCIP/NBT solution (5-bromo-4-chloro-3-indoyl phosphate/nitro blue tetrazolium; Sigma) for 5 minutes to detect the proteins reacted with patients' serum.

In order to minimize technical errors in detection rate in each test, each test comprised a positive standard serum and negative control serum. The results of the tests were assessed by at least two investigators independently with naked eyes. When test serum showed strong band to certain airway epithelial cell protein compared to negative control serum, the stained degree of the band were the same as or stronger than that of positive standard serum, and the result read by the two investigators corresponded to each other, which case was defined as the positive detection of autoantibodies.

Mouse monoclonal antibody to cytokeratin 18 protein (clone CK5, Sigma Chemical Co., St. Louis, MO), goat antibody to human alpha-enolase protein (Santa Cruz Biotechnology. Santa Cruz, CA) and their alkaline phosphatase-conjugated secondary antibodies were included in each experiment to confirm the location of the reacting autoantigen proteins when the detection results of the autoantibodies to airway epithelial cell proteins were read.

The target autoantigen protein was localized by comparing and analyzing the results of immunoblot localization of airway epithelial cell proteins on the PVDF membrane reacting with IgG autoantibodies of patients with bronchial asthma and the coomassie blue staining patterns and molecular weights of airway epithelial proteins on the PVDF membranes transferred after SDS-PAGE. Fig. 1 shows immunoblot anlysis of human airway epithelial cell (A549 cell) proteins reacting with IgG autoantibodies in serum samples of healthy controls and patients with bronchial asthma. Lanes 1 to 3 show the result for serum samples of healthy control, lanes 4 to 9 show the result for serum samples of patients with severe asthma, lane 10 shows the result for goat antibodies to human alpha-enolase, lane 11 shows the result for mouse monoclonal antibodies to cytokeratin 18 protein, lane 12 shows the result of the reaction with dilution buffer only as negative control. The expression '←*' indicates the location of human alpha-enolase proteins confirmed by goat antibodies.

Fig. 1 shows that IgG autoantibodies in each subject's serum samples are negative in 3 healthy controls and are positive in 5 of 6 patients with severe asthma.

Also, Fig. 2 shows the result of staining human airway epithelial cell proteins with coomassie blue after SDS-PAGE. Lane 1 shows the result for recombinant human cytokeratin 18 protein and lane 2 shows the result for the proteins extracted from airway epithelial cells (A549). The location of autoantigen protein was marked in Fig. 2 by comparing with the location of airway epithelial cell proteins reacting with IgG antibodies in serum from patients with severe asthma as in Fig 1. The expression '←*' indicates the location of 52-kDa autoantigen protein reacting with IgG antibodies in serum samples from patients with severe asthma.

As a result, it was confirmed that there was airway epithelial cell autoantigen reacting to IgG antibodies in serum samples of the patients with severe asthma, and the molecular weight of the autoantigen was about 52-kDa.

(1-4) Detection of airway epithelial cell autoantigen in patients with bronchial asthma

It was confirmed whether antoantibodies to airway epithelial cell autoantigen were present or not in serum samples of 78 patients with bronchial asthma and 58 healthy controls of the above (1-1), accrording to the method in (1-3).

As a result, autoantibodies to 52-kDa bronchial epithelial cell protein were detected in serum samples of 32 of 78 patients with bronchial asthma (severe asthma) (41%). This showed significantly higher detection rate of IgG autoantibodies than that of healthy control (2 of 58 healthy control, 3%) (chi-square test, $p<0.001$).

Table 1 shows comparision of the detection rate of IgG autoantibodies to 52-kDa airway epithelial cell protein in serum samples of healty controls and patients with bronchial asthma.

[Table 1]

| Groups | Number | Detection rate of IgGautoantibodies to 52-kDa protein (%) | p value* |
|---|---|---|---|
| Healthy controls | 58 | 2(3%) | < 0.001 |
| Patients with bronchial asthma (patients with severe asthma) | 78 | 32 (41%) | |
| * The statistical significance between the two groups was calculated by chi-square test. | | | |

As shown in the above, it was confirmed that patients with bronchial asthma had significantly higher detection rate of IgG autoantibodies to 52-kDa airway epithelial cell protein compared with that of heathy controls.

(1-5) Identification of autoantigen protein reacted with serum samples of patients with bronchial asthma

Airway epithelial cell 52-kDa autoantigen protein reacting with IgG autoantibodies in serum samples of the above patients with severe asthma was identified using the following methods:

The airway epithelial cell proteins were separated by SDS-PAGE, and then stained by coomassie blue (Fig. 2). Lane 1 shows the results for the recombinant human cytokeratin 18 protein, and lane 2 shows the results for the airway epithelial cell (A549) proteins. The expression '←*' indicates the location of 52-kDa autoantigen protein reacting with IgG antibodies in serum samples of patients with severe asthma.

The protein band corresponding to the location of the autoantigen protein was excised from gel, and the excised gel slice was digested with trypsin (enzymatic in-gel digestion).

Trypsin-digested peptide fragments were subject to LC-MS/MS (liquid chromatography-electrospray tandem mass spectrometry) analysis using QTOF2 (Quadrupole time-of-flight mass spectrometry, Micro mass, Beverly, MA) instrument.

Protein identification was accomplished with the Mascot program (Matrix Science. U.K.) using NCBI (Besthesda, MD) non-redundant protein database.

52-kDa airway epithelial cell autoantigen protein was treated with tyripsin to obtain the digested peptide fragments, and then mass and amino acids sequence of the peptide fragments were analyzed. As a result, it was proven that the analyzed 9 peptides corresponded to those of human alpha-enolase proteins in mass and amino acids sequence of peptides. Thus, it was identified that 52-kDa autoantigen protein was human alpha-enolase (Table 2).

[Table 2]

| Matching peptide no. | Mass (Mr) measured | Peptide sequences (+modifications) |
|---|---|---|
| 1 | 898.22 | TIAPALVSK |
| 2 | 1142.17 | IGAEVYHNLK |
| 3 | 1311.03 | LMIEMDGTENK (+2 Oxidations of Methionine) |
| 4 | 1405.14 | GNPTVEVDLFTSK |
| 5 | 1424.15 | YISPDQLADLYK |
| 6 | 1555.13 | VVIGMDVAASEFFR (+ Oxidation of Methionine) |
| 7 | 1803.21 | AAVPSGASTGIYEALELR |
| 8 | 1959.14 | DATNVGDEGGFAPNILENK |
| 9 | 2031.23 | FTASAGIQVVGDDLTVTNPK |

(1-6) Reconfirmation of alpha-enolase as autoantigen

In order to reconfirm that the above-identified 52-kDa autoantigen was alpha-enolase, 2-dimensional immunoblot analysis was performed. To prepare airway epithelial cell proteins to use in 2-dimensional immunoblot analysis, the human airway epithelial cells (A549 cell) were directly lysised in loading buffer containing 9M urea as recommended by the manufacturing company of IPG strip (immobilized pH gradient strip; Amersham Pharmacia Biotech, Piscataway, NJ). For 2-dimensional immunoblot analysis, IEF (isoelectrical focusing) was performed using linear immobilized pH gradient strip (Amersham Pharmacia Biotech, Piscataway, NJ), and then SDS-PAGE and immunoblotting was performed as described in the above. The above proteins were reacted with IgG autoantibodies in serum from patients with severe asthma or goat antibodies to human alpha-enolase (Santa Cruz Biotech Inc., Santa Cruz, CA).

As secondary antibody-conjugates, alkaline phosphatase-conjugated goat anti-human IgG antibodies or rabbit anti-goat antibodies (Sigma Chemical Co., St. Louis, MO) were reacted to those. After the reaction, antibody-binding proteins were stained by using BCIP/NBT as described in the above.

Fig. 3 shows 2-dimensional immunoblot analysis of human airway epithelial cell proteins reacting with IgG autoantibodies in serum samples of patients with severe asthma. The autoantigen had 52-kDa molarcular weight and isoelectrical point (pI value) was about 7 (Fig. 3).

Fig. 4 shows 2-dimensional immunoblot localization of alpha-enolase proteins in airway epithelial cell proteins using goat antibody to human alpha-enolase. From the results of immunoblot analysis, it was confirmed that the alpha-enolase proteins have the same molarcular weight and isoelectrical point (pI value) as autoantigen reacting with IgG autoantibodies in serum samples of patients with severe asthma. Thus, it was reconfirmed that human airway epithelial cell autoantigen reacting with IgG autoantibodies in serum samples of patients with severe asthma was alpha-enolase protein.

(1-7) Detection of autoantibodies in blood of patients with severe asthma using recombinant human alpha-enolase protein

According to the previously reported method (Lee KH, et al., Arthritis Rheum. 2003;48:2025-2035), cDNA of human alpha-enolase proteins were transfected to E.coli. From the transfected E.coli, human alpha-enolase proteins were synthesized and isolated and purified.

By using the above recombinant human alpha-enolase proteins, it was confirmed whether autoantibodies reacting with this protein were detected in serum samples of patients with severe asthma by immunoblot analysis.

Fig. 5 shows immunoblot detection of IgG autoantibodies to recombinant human alpha-enolase in serum samples of healthy controls and patients with severe asthma. Lanes 1 to 3 show the results for serum samples of healthy controls, lanes 4 to 9 show the results for serum samples from patients with severe asthma, lane 10 shows the results for goat antibodies to human alpha-enolase, lane 11 shows the result for mouse monoclonal antibodies to cytokeratin 18 proteins, and lane 12 shows the result for dilution buffer only as a negative control.

The expression '←*' indicates the location of human alpha-enolase proteins confirmed by goat antibodies. The serum samples used in Fig. 5 and their arrangement orders were the same as those of Fig. 1.

Fig. 5 shows that IgG autoantibodies in each subject's serum were negative in 3 persons among healthy controls and were positive in 5 among 6 patients with severe asthma.

Such result remarkably corresponded to the detection result of IgG autoantibodies to 52-kDa autoantigen using airway epithelial cell proteins in Fig. 1. Thus, it was reconfirmed that 52-kDa airway epithelial cell autoantigen was human alpha-enolase protein (Fig. 5).

(1-8) Detection of IgG autoantibodies to alpha-enolase in aspirin-hypersensitive asthma

By using immunoblot methods described in (1-3) and (1-7), it was confirmed whether IgG autoantibodies to alpha-enolase were detected in serum samples of 22 asthmatic patients with asprin-hypersensitivity and 139 asthmatic patients without history of hypersensitivity for aspirin or other non-steroidal anti-immflamatory drugs, where the patients were classified according to the criterion in (1-1).

As a result, IgG autoantibodies to alpha-enolase were detected in the serum samples of 13 (59.1%) patients among 22 asthmatic patients with asprin-hypersensitivity and were detected in the serum samples of 28 (20.1 %) patients among 139 asthmatic patients without history of hypersensitivity for aspirin or other non-steroidal anti-immflamatory drugs. These results show significantly higher detection rates than healthy controls (3%; 2 of 58 healty control; Table 1) ($p<0.05$). Also, it could be recognized that asthmatic patients with aspirin-hypersensitivity (asprin-hypersensitive asthmatic patients) shows significantly higher detection rate of IgG autoantibodies to alpha-enolase than healthy controls or asthmatic patients without aspirin-hypersensitivity ($p<0.05$). Such result shows that detection of IgG autoantibodies to alpha-enolase could predict the presence of asprin-hypersensitivity in asthmatic patients.

**[EXAMPLE 2] Confirmation of IgG autoantibodies to alpha-enolase protein in serum samples of patients with chronic obstructive pulmonary disease**

(2-1) Subjects

The inventors examined serum samples obtained from 9 patients with chronic obstructive pulmonary disease, 2 patients with emphysema showing normal pulmonary function and 58 healthy controls. Diagnosis of chronic obstructive pulmonary disease was determined by recently reported criterion of NHLBI/WHO GOLD Workshop summary (Am J Respir Crit Care Med 2001;163:1256-1276). Both of 2 patients with emphysema had smoking history of more than 10 pack years. They have been clinically complained dyspnea symptom and chest X-ray showed findings compatible with emphysema. However, they had normal findings with values of FEV1 (forced expiratory volume in one second) and FVC (forced vital capacity) were greater than 80% of predictive value on pulmonary function test. All 9 patients with chronic obstructive pulmonary disease had clinical histories compatible with chronic obstructive pulmonary disease such as cough, sputum, dyspnea, etc. Also, their FEV1 measured following the inhalation of bronchodilator showed less than 80% of predictive value on pulmonary function test, and the ratio of FEV1/FVC was less than 70%. All patients with chronic obstructive pulmonary disease had smoking history of more than 10 pack years, but had no signs of tuberculosis or other pulmonary diseases in chest X-ray. The serum samples of the patients with chronic obstructive pulmonary disease were stored at -20 °C.

(2-2) Extraction of cell protains and immunoblot analysis

In order to confirm human airway epithelial cell proteins which bind with IgG autoantibodies in the serum of patients with chronic obstructive pulmonary disease, immunoblot analysis was performed as shown in (1-3) using human airway epithelial cells (1-2) and the serum samples of patients with chronic obstructive pulmonary disease (2-1).

Fig. 6 shows immunoblot anlysis of human airway epithelial cell (A549 cell) proteins reacting with IgG autoantibodies in serum samples of healthy controls, patients with emphysema showing normal pulmonary function, and patients with chronic obstructive pulmonary disease. Lanes 1 and 2 show the results for serum samples of healthy controls, lanes 3 & 4 show the results for serum samples of patients with emphysema showing normal pulmonary function, lanes 5-13 show the results for serum samples of patients with chronic obstructive pulmonary disease, lane 14 shows the result for a serum sample of a patient with bronchial asthma. lane 15 shows the result for mouse monoclonal antibodies to cytokeratin 18 protein, and lane 16 shows the result for goat antibodies to alpha-enolase.

Fig. 6 shows that IgG autoantibodies to 52-kDa airway epithelial cell protein were detected in 4 (44.4%) of 9 patients with chronic obstructive pulmonary disease. This result showed significantly higher detection rate than that of healthy controls (3.4%: 2 of 58 healthy controls; chi-square test, $p<0.05$). Also, it was confirmed that 52-kDa airway epithelial cell autoantigens reacting with IgG autoantibodies in the serum from patients with chronic obstructive pulmonary disease corresponded to the position of alpha-enolase checked by anti-human alpha-enolase antibodies (Fig. 6).

Also. IgG autoantibodies to protein band corresponding to cytokeratin 18 protein checked by mouse monoclonal antibodies were detected in 8 (89%) of total 9 patients with chronic obstructive pulmonary disease. This result showed significantly higher detection rate than that of healty controls (5 of 58 healthy control; 8.6%) (chi-square test. $p<0.05$) (Fig. 6).

(2-3) Detection of autoantibodies in blood of patients with chronic obstructive pulmonary disease using recombinant human alpha-enolase protein

By using the above subjects' serum of (2-2) and human recombinant alpha-enolase proteins produced by the genetic engineering technology of (1-7), immunoblot was performed in same sample arrangement as in Fig 6. The results are shown in Fig. 7.

Fig. 7 shows immunoblot detection of IgG autoantibodies to recombinant human alpha-enolase protein using serum samples of healthy controls, patients with emphysema showing normal pulmonary function, and patients with chronic obstructive pulmonary disease. Lanes 1 and 2 show the results for serum samples of healthy controls, lanes 3 and 4 show the results for serum samples of patients with emphysema showing normal pulmonary function, lanes 5-13 show the results for serum samples of patients with chronic obstructive pulmonary disease, and lane 14 shows the result for a serum sample of a patient with asthma.

As shown in Fig. 7, IgG autoantibodies to recombinant human alpha-enolase proteins were detected in 4 (44.4%) of 9 patients with chronic obstructive pulmonary disease. This detection rate was significantly higher than that of healthy controls (4 of 58 healthy control, 6.9%) (chi-square test, $p<0.05$).

As shown in Fig. 6 and Fig. 7, in case of patients with chronic obstructive pulmonary disease, IgG autoantibodies were detected by recombinant alpha-enolase protein or alpha-enolase protein in airway epithelial cells.

Thus, it was recomfirmed that the autoantigen reacting with IgG autoantibodies in the serum of patients with chronic obstructive pulmonary disease was alpha-enolase protein (Fig. 7).

**[EXAMPLE 3] Detection of IgG autoantibodies to recombinant human alpha-enolase protein in serum samples of patients with chronic inflammatory airway diseases by enzyme-linked immunosorbent assay**

By enzyme-linked immunosorbent assay, IgG autoantibodies to recombinant human alpha-enolase proteins were detected in serum samples of patients with bronchial asthma and patients with chronic obstructive pulmonary disease.

The human alpha-enolase proteins produced by the above genetic engineering technology were diluted with 0.1M carbonate buffer (pH 9.6) in 5 μg/mℓ and put in 96-well microtiter plate at 50 μℓ/well, then reacted for 16 hours at 4 °C.

After the reaction, it was washed with phosphate buffer saline containing 0.05% Tween 20 (PBST) in three times, and PBST containging 10% bovine serum (10% BS-PBST) was put in wells at 350 μℓ/well, then reacted for 1 hour at rom temperature.

Each of patients' serums to be tested (each of 2 patients from the groups of healthy control, patients with severe asthma and patients with chronic obstructive pulmonary disease) were diluted with 10% BS-PBST (at the ratios of 1:20, 1:40 and 1:80 v/v), then put in wells at 50 μℓ/well, and reacted for 2 hours at room temperature. It was washed again with PBST three times, and then alkaline phosphatase-conjugated goat anti-human IgG antibodies (Sigma) were put in the wells at 50 μℓ/well and reacted for 2 hours. After washing the wells three times, 100 μℓ of p-nitrophenylphosphate(pNPP)-colorizing solution (Sigma Chemical Co, Saint Louis, MO, USA) was added into each of wells. After 10 minites, the reaction was stopped by adding 100 μℓ of 0.1 N NaOH solution into each of wells, and then absorbance values of the wells were measured with a microplate leader.

The result of measurement was expressed in terms of corrected absorbance value (mean value of absobance obtained by reacting each of diluted samples with wells containing human alpha-enolase proteins - mean value of absobance obtained by reacting the same samples with wells containing only carbonate buffer without antigen protein.

The tests were performed in quadruplicate for each of test samples, and the results were expressed in terms of mean and standard deviation.

Fig. 8 shows the result detecting the reaction between recombinant human alpha-enolase protein and IgG autoantibodies in serum samples of 2 healthy controls (control 1 and control 2) and 2 patients with severe asthma (patient 1 and patient 2) and 2 patients with chronic obstructive pulmonary diseases (patient 3 and patient 4) by enzyme-linked immunosorbent assay.

As a result, the absorbances of 2 patients with severe asthma and 2 patients with chronic obstructive pulmonary diseases were significantly higher than those of 2 healthy controls (t-test, $p < 0.05$) (Fig. 8).

**[Example 4] Immunoblot analysis for mouse alpha-enolase protein**

In order to determine whether IgG autoantibodies in serum samples of patients with severe asthma and patients with chronic obstructive pulmonary disease are selectively reacted with human alpha-enolase proteins or they could react with alpha-enolase proteins of other mammal cells, the inventors performed immunoblot analysis by using mouse liver cancer cell line, Hepa 1-6.

Mouse liver cancer cell line, Hepa 1-6 cell line (ATCC CRL-1830; VA, USA; Darlington GJ et al, J Nat'l Cancer Inst 1980;64:809-819) were commercially obtained, and then cultured by ATCC's recommendation. Then, Hepa 1-6 cell proteins were obtained in the above extraction method of (1-3).

Immunoblot analysis was performed with the proteins of human airway epithelial cell (A549) and mouse hepatoma line (Hepa 1-6) by using serum samples from 2 patients with severe asthma, according to the method of (1-3).

Fig. 9 shows immunoblot analysis of human airway epithelial cell (A549) and mouse hepatoma cell (Hepa 1-6) proteins reacting with IgG autoantibodies in serum samples of two patients with severe asthma. Lanes 1, 3 and 5 show the results of electrophoresis of the cultured human airway epithelial cell proteins, and lanes 2, 4 and 6 show the results of electrophoresis of mouse hepatoma cell proteins. Lanes 1 & 2 and lanes 3 & 4 show the result using different serum samples of two patients with severe asthma, and lanes 5 and 6 show the result of reacting with goat antibodies to human alpha-enolase.

From the results obtained by using the serum samples of patients with severe asthma, it was confirmed that IgG autoantibodies to alpha-enolase in their serum samples could react with mouse alpha-enolase proteins localized by goat antibodies and mouse alpha-enolase proteins had the same molarcular weight of human alpha-enolase proteins. Such results are regarded to be compatible to prior report that human alpha-enolase proteins (SEQ ID NO: 1) and mouse alpha-enolase proteins (SEQ ID NO: 2) consist of total 434 amino acids, and they have a very high homology in base sequence, as known by prior document (Pancholi V., Cell Mol Life Sci. 2001;58:902-920) (Fig. 9).

As shown in the above, the test results of autoantigen-autoantibodies using human airway epithelial cell and mouse liver cell corresponded to each other. Thus, it was confirmed that mouse alpha-enolase protein could be used to detect autoantibodie in serum samples of patients with severe asthma and patients with chronic obstructive pulmonary disease.

**[Example 5] Inhibition of IgG antibody-induced cytotoxicity to airway epithelial cells by the administration of alpha-enolase protein**

(5-1) Confirmation of cytotoxicity to airway epithelial cells by IgG autoantibodies

Cytotoxicity to airway epithelial cell by IgG autoantibodies was measured by the microcytotoxicity method using Terasaki tray and modifying prior reported method (Martin S, et al; Tissue Antigens 1991;37:152-155).

In the above test, IgG antibodies were isolated from plasma of 2 patients with severe asthma. 2 patients with chronic obstructive pulmonary disease, and a healthy control, where patients with severe asthma and chronic obstructive pulmonary disease had IgG autoantibodies to human alpha-enolase protein.

IgG antibodies of 2 patients with severe asthma and 1 healthy control were isolated to have a purity of 90%, by using ethanol precipitation and ultrafiltration according to prior preparing method of IgG antibodies (Lebing W et al. Vox Sang 2003:84:193-201). IgG antibodies of 2 patients with chronic obstructive pulmonary disease were isolated in purity of greater than 95% by affinity chromatography using Protein A column. The purity of the isolated IgG antibodies was determined by SDS-PAGE and protein staining. Also, the amount of endotoxin in the isolated IgG antibody solution was measured quantitatively by using LAL (Limulus amebocyte lysate). As a result, the contamination of endotoxin was not detected.

Two kinds of human IgG antibodies used for intravenous administration (Livgamma. Dongshin Pharmaceutical Co., Korea; IVglobulin, Green Cross Pharmaceutical Co., Korea) were commercially obtained and used as control IgG antibodies, where the control IgG antibodies were isolated from plasma of a large mumber of blood donors, to have a purity of more than 95%.

First of all, the above human IgG antibodies were diluted by using DMEM/F12 medium at concentrations of 5mg/mℓ, 1mg/mℓ, and 0.2mg/mℓ. Then, the diluted samples were put in 96-well Terasaki tray at a ratio of 1 μℓ/well in quadruplicate. Also, samples containing only DMEM/F12 medium were put in 96-well Terasaki tray in quadruplicate as negative controls.

The cultured human airway epithelial cell line A549 was treated with Trypsin/EDTA and isolated, then 1 μℓ (2000 cells/μℓ) of the solution was put in each wells. In order to prevent evaporation of the solution, 5 μℓ of mineral oil was put in the wells. And then, the wells were reacted for 2 hours and 30 minutes. 5% eosin Y dye (Sigma Chemical Co.) was put in the wells at a ratio of 5 μℓ/well to stain the cells. Then, formalin solution was put in the wells at a ratio of 5 μℓ/well to fix the stained cells. A coverslide was covered and the number of cells without damage of cell membrane was counted by light microscope to determine cytotoxicity.

The cytotoxicity to airway epithelial cell by IgG antibodies was expressed as cytolysis % according to following equation, by comparing mean number of cells in wells reacting with the samples and mean number of cells in negative control wells containing only DMEM/F12 medium.

【Equation 1】

Cytotoxicity (cytolysis %) = [(mean number of cells in negative control wells - mean ·number of cells in wells containg samples) / mean number of cells in negative control wells] x 100

According to the same method as in the above, cytotoxicity to airway epithelial cell was measured by goat IgG antibodies to alpha-enolase protein (Santa Cruz Biotechnology, Santa Cruz, CA) as the positive control test and normal goat IgG antibodies (reagent grade; Sigma Chemical Co.. St. Louis. MO) as the negative control antibodies.

The result of the test was obtained in quadruplicate to each sample, and then expressed in terms of mean and standard deviation.

Fig. 10a and Fig. 10b show that IgG antibodies isolated from plasma samples of 2 patients with severe asthma (patient 1, patient 2) and 2 patients with chronic obstructive pulmonary disease (patient 3, patient 4) show significantly higher cytotoxicity than that of IgG antibodies isolated from plasma of a healthy control (healthy control 1), Livgamma (healthy controls 2), or IVglobulin (healthy controls 3) under the conditions of 1 μg/well and 5 μg/well (t-test, $p < 0.05$), where Livgamma and IVglobulin are commercial IgG antibodies used for intravenous administration. Fig. 10c shows that goat IgG antibodies to alpha-enolase protein (specific IgG) show significantly higher cytotoxicity to airway epithelial cell, than that of normal goat antibodies (control IgG) under all of the conditions of 12.5 ng/well to 100 ng/well (t-test, p < 0.05).

(5-2) Inhibition of cytotoxicity to airway epithelial cell by IgG autoantibodies

In order to absorb autoantibodies to alpha-enolase in serum samples of patients with chronic inflammantory airway diseases, the recombinant human alpha-enolase protein prepared in the method of (1-6) or bovine serum albumin were coupled to agarose beads (Sepharose 4B™ bead, Amersham Pharmacia Biotech, Piscataway, NJ) at a ratio of 1 mg protein per 1 mℓ of agarose beads as recommended by manufacturer of agarose bead.

100 μℓ each of the above 2 kinds of beads was mixed with 100 μg of IgG antibodies which were isolated from serum samples of 2 patients with severe asthma and diluted in DMEM/F12 medium to have a concentration of 5 mg/mℓ, and then, the mixture was reacted for 16 hours at 4°C. After the reaction, supernatant was collected by centrifugation, and the cytotoxicity to human airway epithelial cell by IgG antibodies was measured in the same method as the above.

Glutathione-S-transferase (GST)-conjugated recombinant human alpha-enolase proteins or GST alone was prepared in the method in (1-6) and transferred to PVDF membrane. PVDF membrane strips containing 50 μg of recombinant human alpha-enolase protein or 50 μg of GST protein were cut tinily and put in eppendorf tube. Then, 50 μg of IgG antibodies of 1 patient with chronic obstructive pulmonary disease which was diluted with DMEM/F12 medium to have a concentration of 5mg/mℓ and was added in eppendorf tubes. After the addition, the mixture was reacted for 16 hours at 4°C. After the reaction, supernatant was collected by centrifugation and used in the experiment.

The result of the experiment was obtained in quadruplicate to each samples, and then, expressed in terms of mean and standard deviation.

Fig. 11 shows that when the IgG antibodies of patients with severe asthma (patient 1, 2) and patients with chronic obstructive pulmonary disease (patient 3) were adsorbed with recombinant human alpha-enolase protein prior to the addition to airway epithelial cells, the cytotoxicity to airway epithelial cell by IgG autoantibodies (shown as "B" in each graph) was significantly reduced compared to the case of adsorbtion with the same amount of control antigens, bovine serum albumin or GST protein (shown as "A" in each graph) (t-test, $p < 0.05$).

The following Table 3 shows the results which represent cytotoxicity to airway epithelial cell as mean ± standard deviation, when IgG antibodies of two patients with severe asthma (patient 1, 2) and a patient with chronic obstructive pulmonary disease (patient 3) are absorbed with alpha-enolase protein, or with control antigen.

From Table 3, it was confirmed that when IgG antibodies were absorbed with alpha-enolase proteins, cytolysis % was significantly lower compared to the case of the absorption with control proteins.

[TABLE 3]

| Patients | Absorption with control proteins (cytolysis %) | Absorption with alpha-enolase proteins (cytolysis %) |
|---|---|---|
| Patient 1 | 73.9 ± 2.8* | 9.4 ± 4.9 |
| Patient 2 | 38.8 ± 5.6* | 4.9 ± 4.5 |
| Patient 3 | 41.5 ± 9.7* | 14.9 ± 13.5 |
| *: P<0.01 | | |

Such result shows that alpha-enolase proteins can be used for the inhibition of cytotoxic reaction to airway epithelial cell induced by IgG autoantibodies to alpha-enolase protein in the blood of patients with severe asthma or patients with chronic obstructive pulmonary disease.

**[Example 6] Confirmation of diagnosability enhancement of chronic inflammantory airway diseases by simultaneous measurement of IgG autoantibodies to alpha-enolase and cytokeratin 18**

The presence of autoantibodies to alpha-enolase and cytokeratin 18 which were proved as autoantigen of bronchial asthma and chronic obstructive pulmonary disease was confirmed in patients with bronchial asthma (patients with severe asthma), patients with chronic obstructive pulmonary disease and non-smoking healthy control.

The proteins of airway epithelial cell (A549) and the immunoblot analysis method described in the above (1-3) were used. The immunoblot analysis was performed by using serums from 78 patients with severe asthma. 9 patients with chronic obstructive pulmonary disease and 58 non-smoking healthy controls.

Goat anti-human alpha-enolase antibodies (Santa Cruz Biotechnology, Santa Cruz, CA) were used as antibodies to alpha-enolase, and anti-human cytokeratin 18 monoclonal antibodies (Clone CK5, Sigma chemical Co., st. Louis, MO) were used as antibodies to cytokeratin 18.

Table 3 shows the result for immunoblot analysis of IgG autoantibodies to alpha-enolase protein of airway epitheial cell, cytokeratin 18 protein of airway epitheial cell and recombinant alpha-enolase protein in serums samples of patients with severe asthma and healthy control.

From the result, when the presence of IgG autoantibodies to alpha-enolase was examined, patients with severe asthma show significantly higher detection rate ($p<0.05$) than that of healthy controls.

Also, when the presence of IgG autoantibodies to alpha-enolase and cytokeratin 18 was examined simultaneously to consider the result over all, the detection rate of IgG autoantibodies for one or more of the above two autoantigen proteins was significantly higher in patients with severe asthma($p<0.05$) than healthy controls (Table 4).

The Table 4 also shows that when IgG autoantibodies to alpha-enolase and cytokeratin 18 were measured simultaneously and combined the results, the diagnostic sensitivity for severe asthma was 21.8% higher than that of the case of diagnosing severe asthma only by IgG autoantibodies to alpha-enolase (Table 4).

[TABLE 4]

| | Number of patients (%) | |
|---|---|---|
| Detection rate of IgG autoantibodies | Severe asthma (n=78) | Healthy controls (n=58) |
| Alpha-enolase | 32 (41.0)* | 2 (3.4) |
| Cytokeratin 18 | 25 (32.1)* | 5 (8.6) |
| Alpha-enolase +cytokeratin18‡ | 49 (62.8)* | 7 (12.1) |
| Recombinant alpha-enolase | 31 (39.7)* | 4 (6.9) |
| *P<0.01 for the comparison with healthy controls.<br>‡Either of autoantibodies to alpha-enolase or cytokeratin 18 was positive. | | |

As shown in Fig. 6, in case of detecting only IgG autoantibodies alpha-enolase in airway epithelial cells, the diagnostic sensitivity of chronic obstructive pulmonary diseases was 44.4 % (4 of 9 patients). But all 9 patients with chronic obstructive pulmonary diseases (100%) showed IgG autoantibodies to one or more of alpha-enolase protein and cytokeratin 18 protein. This result shows a significantly higher detection rate than those in 58 healthy controls (12.1%) ($p<0.05$) (Table 4).

Thus, it can be recognized that detecting the presence of IgG autoantibodies to alpha-enolase protein and cytokeratin 18 protein simultaneously is useful to diagnose and classify patients with severe asthma and patients with chronic obstructive pulmonary disease.

The presence of atopy was detected in 161 patients with bronchial asthma by the skin prick test using 50 kinds of common allergen (Bencard Co. Brentford, UK), and the presence of aspirin-hypersensitivity was confirmed as (1-1).

In the above classified patients with atopy and patients with aspirin-hypersensitivity, the presence of autoantibodies to alpha-enolase and/or cytokeratin 18 was determined.

As shown in Table 5. when the presence of autoantibodies to alpha-enolase was measured, asthmatic patients with aspirin-hypersensitivity (or refferred to "patients with aspirin-hypersensitive asthma") showed a significantly higher dectection rate than that of patients without aspirin-hypersensitivity ($p<0.05$).

Also, when the presence of autoantibodies to alpha-enolase and cytokeratin 18 was detected simutaneously, patients with aspirin-hypersensitive asthma showed a significantly higher dectection rate of IgG autoantibodies to one or more of the above two autoantigen proteins than that of asthmatic patients without aspirin-hypersensitivity ($p<0.05$) (Table 5).

Also, when the two autoantibodies were detected simultaneously, diagnosability for aspirin-hypersensitive asthma was enhanced by about 18.2%, comparing the case of detecting IgG autoantibodies to alpha-enolase autoantigen protein only (Table 5).

[Table 5]

| Relationship between the presence of atopy or aspirin-hypersensitivity and detection rate of IgG autoantibodies to airway epithelial cell autoantigen proteins in patients with bronchial asthma | | | | |
|---|---|---|---|---|
| | Atopy | | Aspirin-hypersensitivity | |
| | Negative | Positive | Negative | Positive |
| Number of patients | 49 | 112 | 139 | 22 |
| | | | | |

(continued)

| Detection rate of IgG autoantibodies | Number of patients (%) | | Number of patients (%) | |
|---|---|---|---|---|
| Alpha-enolase | 15 (30.6) | 26 (23.2) | 28 (20.1) | 13 (59.1 )* |
| Cytokeratin 18 | 20 (40.8)† | 28 (25.0) | 41 (29.5) | 7 (31.8) |
| Alpha-enolase + Cytokeratin 18‡ | 30 (61.2)† | 47 (42.0) | 60 (43.2) | 17 (77.3)§ |
| Recombinant alpha-enolase | 14 (28.6) | 29 (25.9) | 33 (23.7) | 10 (45.5)¶ |

*P<0.001 for the comparison with asthmatic patients without aspirin-hypersensitivity.
†P=0.04 for the comparison with atopic asthmatic patients.
‡Either of autoantibodies to alpha-enolase or cytokeratin 18 was positive.
§P=0.003 for the comparison with asthmatic patients without aspirin-hypersensitivity.
¶P=0.03 for the comparison with asthmatic patients without aspirin-hypersensitivity.

Thus, it can be confirmed that detection of the presence of autoantibodies to both alpha-enolase and cytokeratin 18 simultaneously is useful to diagnose asthmatic patients with aspirin-hypersensitivity with a higher diagnostic sensitivity.

**[Example 7] Confirmaton for inhibiting secretion of proinflammatory cytokine IL-8 (interleukin-8) in patients with bronchial asthma by the composition comprising alpha-enolase of the present invention**

In prior report, it was known that the administration of IL-8 to guinea pig causes airway hyperresponsiveness which is a common characteristics of bronchial asthma and chronic obstructive pulmonary disease, as well as neutrophilic inflammation of lower airway (Xiu Q et al., Clin Exp Allergy 1995;25:51-9). It was also reported that when monoclonal antibodies to IL-8 were administrated to patients with chronic obstructive pulmonary disease to neutralize action of IL-8, dyspnea symptom was significantly improved (Mahler DA, Chest 2004;126:926-934). It was also known that monoclonal antibodies to IL-8 could be used for treating bronchial asthma (U.S. Pat. No. 5,874,080).

Based on such prior reports, the amount of secretion of IL-8 by autoantibodies of patients with bronchial asthma was monitored. Also, when autoantibodies of patients with bronchial asthma were treated with alpha-enolase of the present invention, the change in the amount of secretion of IL-8 was determined.

(7-1) Secretion of proinflammatory cytokine IL-8 by IgG autoantibodies of patients with bronchial asthma

Human airway epithelial cells (A549) treated with Trypsin/EDTA were put in 96-wells culture plate and diluted with 100 $\mu\ell$ medium per well to be 20,000 cells/well, then adhered for 16 hours in an incubator.

The culture medium was removed from the wells. IgG antibodies of 1 patient with bronchial asthma (Patient I, who was confirmed to have IgG autoantibodies to alpha-enolase in serum sample by immunoblot) were isolated according to the method in (5-1) and commercial IgG antibodies purified from multiple healthy donors (Livgamma, Dongshin Pharmaceutical Co., Korea) were also used. The isolated IgG antibodies were diluted with DMEM/F12 medium, and then put in the wells at a ratio of 200 $\mu\ell$/well in quadruplicate. As control tests, 200 $\mu\ell$ of DMEM/F12 medium or DMEM/F12 medium containing 1% Triton X-100 was put in the wells in quadruplicate and then reacted in an incubator for 16 hours. The above 96-wells culture plates were centrifuged and supernants were collected from each of wells. The concentration of IL-8 in supernant of the culture medium was measured by using ELISA kit (Endogen, Woburn, MA). The result is shown in Fig. 12a.

From Fig. 12a, it can be recognized that IgG antibodies of a patient with bronchial asthma (Patient 1) induce significantly higher level of IL-8 secretion than that of healthy controls (Controls 1) at the concentration of 0.1 mg/m$\ell$ to 2.5mg/m$\ell$ of IgG antibodies ($p < 0.05$).

Thus, it could be confirmed that IgG antibodies of a patient with bronchial asthma induce a development of inflammation by stimulation of IL-8 secretion from airway epithelial cells.

(7-2) Inhibition of proinflammatory cytokine (IL-8) secretion caused by autoantibodies of patients with bronchial asthma using alpha-enolase protein of the present invention

IgG antibodies of patient with bronchial asthma (Patient 1) were diluted to have a concentration of 2.5mg/m$\ell$,

and absorbed for 16 hours with agarose beads which were conjugated with human alpha-enolase prepared by recombinant genetic engineering technique or human serum albumin (a negative control protein) as described in (5-2). Then, supernants were collected, and human airway epithelial cells (A549) were treated with these supernants and reacted in the same method as in (7-1), and the amounts of IL-8 in the culture medium of airway epithelial cells were measured.

From the result of Fig. 12, it can be confirmed that when IgG antibodies of a patient with bronchial asthma (Patient 1) are absorbed with alpha-enolase proteins (B), IL-8 secretion from airway epithelial cell caused by IgG antibodies can be significantly inhibited, compared to the case of absorption with human serum albumin (A) ($p < 0.05$).

The above results of experiments show that IgG autoantibodies to alpha-enolase protein in the blood of a patient with bronchial asthma can enhance the secretion of IL-8 from airway epithelial cells, and alpha-enolase proteins can inhibit the secretion of IL-8 from airway epithelial cells caused by IgG autoantibodies of a patient with bronchial asthma.

Accordingly, it can be recognized that the inflammantory reaction of airway can be repressed by administrating alpha-enolase protein of the present invention into patients with chronic inflammantory airway diseases such as bronchial asthma or chronic obstructive pulmonary disease, thereby inhibiting IL-8 secretion from airway epithelial cells caused by IgG autoantibodies.

## INDUSTRIAL APPLICATION

As decribed in the above, the present invention identified alpha-enolase protein as an autoantigen recognized by autoantibodies in the serum samples of patients having chonic inflammatory airway diseases such as severe asthma or COPD. Thus, a pharmaceutical composition of the present invention comprising alpha-enolase protein as an acitive ingredient can be used as a medicament for preventing, alleviating, and treating of chronic inflammatory airway diseases such as bronchial asthma or COPD through the inhibition of autoantibody-induced cytotoxicity to airway epithelial cells and inhibition of secretion of proinflammatory cytokine caused by autoantibodies from patients with severe asthma or COPD.

Also, a diagnostic composition comprising alpha-enolase protein according to the present invention can be used for diagnosing chronic inflammatory airway diseases such as bronchial asthma or COPD. Further, a diagnostic composition comprising alpha-enolase and cytokeratin 18 proteins simultaneously according to the present invention can be used for detecting, diagnosing or classifying chronic inflammatory airway diseases such as severe asthma, aspirin-hypersensitive asthma or COPD.

## Claims

1. A pharmaceutical composition for use in preventing or treating chronic inflammatory airway diseases comprising alpha-enolase protein as an active ingredient.

2. The composition of claim 1 for the use according to claim 1, wherein the alpha-enolase protein are originated from mammals selected from the group consisting of human, mouse, rat, rabbit, pig, cow and goat.

3. The composition of claim 2 for the use according to claim 2, wherein the alpha-enolase protein has an amino acid sequence of SEQ ID NO: 1.

4. The composition of claim 2 for the use according to claim 2, wherein the alpha-enolase protein has an amino acid sequence of SEQ ID NO: 2.

5. The composition of claim 1 for the use according to claim 1, wherein one of the chronic inflammatory airway diseases is bronchial asthma or chronic obstructive pulmonary disease.

6. The composition of claim 5 for the use according to claim 5, wherein the bronchial asthma is severe asthma or aspirin-hypersensitive asthma.

7. A method of diagnosing chronic inflammatory airway diseases by using diagnostic compositions containing alpha-enolase protein as an active ingredient comprising the steps of:

   (a) obtaining biological samples of a subject suspected to have chronic inflammatory airway diseases such as bronchial asthma or chronic obstructive pulmonary disease;
   (b) contacting the biological samples with a diagnostic composition for diagnosing chronic inflammatory airway diseases comprising alpha-enolase protein to induce the formation of immune complex; and
   (c) diagnosing said chronic inflammatory airway diseases in the subject by detecting the immune complex to determine the presence of autoantibodies to alpha-enolase protein.

8. The method of claim 7, wherein the alpha-enolase protein is originated from mammals selected from the group consisting of human, mouse, rat, rabbit, pig, cow and goat.

9. The method of claim 8, wherein the alpha-enolase protein has the amino acid sequence of SEQ ID NO: 1.

10. The method of claim 8, wherein the alpha-enolase protein has the amino acid sequence of SEQ ID NO: 2.

11. The method of claim 7, wherein one of the chronic inflammatory airway diseases is bronchial asthma or chronic obstructive pulmonary disease.

12. The method of claim 11, wherein the bronchial asthma is severe asthma or aspirin-hypersensitive asthma.

13. A method of screening a therapeutic agent for chronic inflammatory airway diseases by using acomposition for screening a therapeutic agent for chronic inflammatory airway diseases, comprising one or more of alpha-enolase protein, antibodies to said alpha-enolase protein, or autoantibodies to alpha-enolase obtained from patients having said chronic inflammatory airway diseases, comprising the steps of:

    (a) isolating autoantibodies from biological samples obtained from patients having chronic inflammatory airway diseases such as bronchial asthma or chronic obstructive pulmonary disease;
    (b) contacting test materials with the autoantibodies obtained in the above step (a); and
    (c) selecting a therapeutic agent for chronic inflammatory airway diseases by determining whether the test materials can inhibit binding of the autoantibodies to alpha-enolase protein, cytotoxic effects of the autoantibodies to alpha-enolase protein-expressing cells or secretion of inflammatory mediators including inflammatory cytokine from alpha-enolase protein-expressing cells induced by the autoantibodies.

14. The method of screening according to claim 13, wherein the alpha-enolase protein is originated from mammals selected from the group consisting of human, mouse, rat, rabbit, pig, cow and goat.

**15.** The method of screening according to claim 14, wherein the alpha-enolase protein has the amino acid sequence of SEQ ID NO: 1.

**16.** The method of screening according to claim 14, wherein the alpha-enolase protein has the amino acid sequence of SEQ ID NO: 2.

**17.** The method of screening according to claim 13, wherein one of the chronic inflammatory airway diseases is bronchial asthma or chronic obstructive pulmonary disease.

**18.** The method of any one of claims 13-17, wherein said biological samples are one or more materials selected from the group consisting of blood, plasma, serum, urine, tears, saliva, sputum, nasal secretion, bronchial secretion, bronchial washing fluid, pulmonary secretion and alveolus washing fluid.

**19.** A diagnostic composition for diagnosing chronic inflammatory airway diseases comprising alpha-enolase protein and cytokeratin 18 protein simultaneously.

**20.** The composition of claim 19, wherein the alpha-enolase protein is originated from mammals selected from the group consisting of human, mouse, rat, rabbit, pig, cow and goat.

**21.** The composition of claim 20, wherein the alpha-enolase protein has the amino acid sequence of SEQ ID NO: 1.

**22.** The composition of claim 20, wherein the alpha-enolase protein has the amino acid sequence of SEQ ID NO: 2.

**23.** The composition of claim 19, wherein the cytokeratin 18 protein is originated from mammals selected from the group consisting of human, mouse, rat, rabbit, pig, cow and goat.

**24.** The composition of claim 23, wherein the alpha-enolase protein has the amino acid sequence of SEQ ID NO: 4.

**25.** The composition of claim 19, wherein one of the chronic inflammatory airway diseases is bronchial asthma or chronic obstructive pulmonary disease.

**26.** The composition of claim 25, wherein the bronchial asthma is severe asthma or aspirin-hypersensitive asthma.

**27.** A composition for detecting autoantibodies comprising alpha-enolase protein and cytokeratin 18 protein simultaneously.

**28.** The composition of claim 27, wherein the alpha-enolase protein is originated from mammals selected from the group consisting of human, mouse, rat, rabbit, pig, cow and goat.

**29.** The composition of claim 28, wherein the alpha-enolase protein has the amino acid sequence of SEQ ID NO: 1.

**30.** The composition of claim 28, wherein the alpha-enolase protein has the amino acid sequence of SEQ ID NO: 2.

**31.** The composition of claim 27, wherein the cytokeratin 18 protein is originated from mammals selected from the group consisting of human, mouse, rat, rabbit, pig, cow and goat.

**32.** The composition of claim 31, wherein the alpha-enolase protein has the amino acid sequence of SEQ ID NO: 4.

**33.** A method for detecting autoantibodies by using compositions for detecting autoantibodies containing alpha-enolase protein and cytokeratin 18 protein, comprising the steps of:

(a) obtaining biological samples of a subject;
(b) contacting the biological samples with any one of the compositions of claims 27 to 32 to induce the formation of immune complex; and
(c) detecting the formation of said immune complex in the subject to determine the presence of autoantibodies to alpha-enolase protein and cytokeratin 18 proteins.

**34.** The method of claim 33, wherein the alpha-enolase protein is originated from mammals selected from the group consisting of human, mouse, rat, rabbit, pig, cow and goat.

**35.** The method of claim 34, wherein the alpha-enolase protein has the amino acid sequence of SEQ ID NO: 1.

**36.** The method of claim 34, wherein the alpha-enolase protein has the amino acid sequence of SEQ ID NO: 2.

**37.** The method of claim 33, wherein the cytokeratin 18 protein is originated from mammals selected from the group consisting of human, mouse, rat, rabbit, pig, cow and goat.

**38.** The method of claim 37, wherein the cytokeratin 18 protein have the amino acid sequence of SEQ ID NO: 4.

**39.** The method of claim 33, wherein the subject is a patient having chronic inflammatory diseases.

**40.** A diagnostic composition comprising alpha enolase protein for use in a method for diagnosing chronic inflammatory airway diseases including bronchial asthma and chronic obstructive pulmonary disease by using a diagnostic composition containing alpha-enolase protein, comprising the steps of:

(a) selecting a subject suspected to have chronic inflammatory airway diseases;
(b) injecting intradermally to the subject a diagnostic composition for diagnosing chronic inflammatory airway diseases comprising alpha-enolase protein; and
(c) diagnosing chronic inflammatory airway diseases by examining the injected skin part after 24 to 72 hours of the injection to detect the presence of skin swelling and measure the size of skin swelling to determine the presence of delayed-type hypersensitivity reaction or late-skin reaction to alpha-enolase protein.

**41.** The diagnostic composition for use according to claim 40, wherein the alpha-enolase protein is originated from mammals selected from the group consisting of human, mouse, rat, rabbit, pig, cow and goat.

**42.** The diagnostic composition for use according to claim 41, wherein the alpha-enolase protein has the amino acid sequence of SEQ ID NO: 1.

**43.** The diagnostic composition for use according to claim 41, wherein the alpha-enolase protein has the amino acid sequence of SEQ ID NO: 2.

**44.** The diagnostic composition for use according to claim 40, wherein one of the chronic inflammatory airway diseases is bronchial asthma or chronic obstructive pulmonary disease.

**45.** The diagnostic composition for use according to claim 44, wherein the bronchial asthma is severe asthma or aspirin-hypersensitive asthma.

## Patentansprüche

**1.** Pharmazeutische Zusammensetzung zur Verwendung bei der Prävention oder Behandlung von chronischen entzündlichen Atemwegserkrankungen, die alpha-Enolaseprotein als Wirkstoff umfasst.

**2.** Zusammensetzung gemäss Anspruch 1 zur Verwendung gemäss Anspruch 1, wobei das alpha-Enolaseprotein von Säugern, ausgewählt aus der Gruppe bestehend aus Mensch, Maus, Ratte, Kaninchen, Schwein, Kuh und Ziege, stammt.

**3.** Zusammensetzung gemäss Anspruch 2 zur Verwendung gemäss Anspruch 2, wobei das alpha-Enolaseprotein die Aminosäuresequenz SEQ ID NO: 1 aufweist.

**4.** Zusammensetzung gemäss Anspruch 2 zur Verwendung gemäss Anspruch 2, wobei das alpha-Enolaseprotein die Aminosäuresequenz SEQ ID NO: 2 aufweist.

**5.** Zusammensetzung gemäss Anspruch 1 zur Verwendung gemäss Anspruch 1, wobei eine der chronischen entzündlichen Atemwegserkrankungen Bronchialasthma oder chronisch obstruktive Lungenerkrankung ist.

**6.** Zusammensetzung gemäss Anspruch 5 zur Verwendung gemäss Anspruch 5, wobei das Bronchialasthma schweres Asthma oder Asthma aufgrund von Aspirin-Überempfindlichkeit ist.

**7.** Verfahren zur Diagnose von chronischen entzündlichen Atemwegserkrankungen durch Verwendung diagnostischer Zusammensetzungen, die alpha-Enolaseprotein als Wirkstoff enthalten, umfassend die Schritte:

(a) Gewinnen biologischer Proben von einem Subjekt mit Verdacht auf chronische entzündliche Atemwegserkrankungen, wie Bronchialasthma oder chronisch obstruktive Lungenerkrankung;
(b) In-Kontakt-Bringen der biologischen Proben mit einer diagnostischen Zusammensetzung für die Diagnose von chronischen entzündlichen Atemwegserkrankungen, umfassend alpha-Enolaseprotein, um die Bildung von Immunkomplexen zu induzieren; und
(c) Diagnostizieren der chronischen entzündlichen Atemwegserkrankungen in dem Subjekt durch Detektion des Immunkomplexes, um die Gegenwart von Antikörpern gegen alpha-Enolaseprotein zu bestimmen.

**8.** Verfahren gemäss Anspruch 7, wobei das alpha-Enolaseprotein von Säugern, ausgewählt aus der Gruppe bestehend aus Mensch, Maus, Ratte, Kaninchen, Schwein, Kuh und Ziege, stammt.

**9.** Verfahren gemäss Anspruch 8, wobei das alpha-Enolaseprotein die Aminosäuresequenz SEQ ID NO: 1 aufweist.

**10.** Verfahren gemäss Anspruch 8, wobei das alpha-Enolaseprotein die Aminosäuresequenz SEQ ID NO: 2 aufweist.

**11.** Verfahren gemäss Anspruch 7, wobei eine der chronischen entzündlichen Atemwegserkrankungen Bronchialasthma oder chronisch obstruktive Lungenerkrankung ist.

**12.** Verfahren gemäss Anspruch 11, wobei das Bronchialasthma schweres Asthma oder Asthma aufgrund von Aspirin-Überempfindlichkeit ist.

**13.** Verfahren zum Screenen eines therapeutischen Mittels gegen chronische entzündliche Atemwegserkrankungen durch Verwendung einer Zusammensetzung zum Screenen eines therapeutischen Mittels gegen chronische entzündliche Atemwegserkrankungen, umfassend ein oder mehrere alpha-Enolaseprotein(e), Antikörper gegen das alpha-Enolaseprotein oder Autoantikörper gegen alpha-Enolase, die von Patienten, die an diesen chronischen entzündlichen Atemwegserkrankungen leiden, erhalten werden, umfassend die Schritte:

(a) Isolieren von Autoantikörpern aus biologischen Proben, die von Patienten mit chronischen entzündlichen Atemwegserkrankungen, wie Bronchialasthma oder chronisch obstruktiver Lungenerkrankung, erhalten werden;
(b) In-Kontakt-Bringen der Testmaterialien mit den im vorstehenden Schritt (a) erhaltenen Autoantikörpern; und
(c) Auswählen eines therapeutischen Mittels gegen chronische entzündliche Atemwegserkrankungen durch Bestimmung, ob die Testmaterialien die Bindung der Autoantikörper an alpha-Enolaseprotein, die cytotoxischen Wirkungen der Autoantikörper gegen alpha-Enolaseprotein-exprimierende Zellen oder die Sekretion von Entzündungsmediatoren, einschliesslich Entzündungs-Cytokin aus alpha-Enolaseproteinexprimierenden Zellen, das durch die Autoantikörper induziert wird, inhibieren können.

**14.** Verfahren zum Screenen gemäss Anspruch 13, wobei das alpha-Enolaseprotein von Säugern, ausgewählt aus der Gruppe bestehend aus Mensch, Maus, Ratte, Kaninchen, Schwein, Kuh und Ziege, stammt.

**15.** Verfahren zum Screenen gemäss Anspruch 14, wobei das alpha-Enolaseprotein die Aminosäuresequenz SEQ ID NO: 1 aufweist.

**16.** Verfahren zum Screenen gemäss Anspruch 14, wobei das alpha-Enolaseprotein die Aminosäuresequenz SEQ ID NO: 2 aufweist.

**17.** Verfahren zum Screenen gemäss Anspruch 13, wobei eine der chronischen entzündlichen Atemwegserkrankungen Bronchialasthma oder chronisch obstruktive Lungenerkrankung ist.

**18.** Verfahren gemäss irgendeinem der Ansprüche 13 bis 17, wobei die biologischen Proben ein oder mehrere Material(ien), ausgewählt aus der Gruppe bestehend aus Blut, Plasma, Serum, Urin, Tränen, Speichel, Sputum, Nasensekret, Bronchialsekret, Bronchial-Waschflüssigkeit, Lungenbläschensekret und alveole Waschflüssigkeit, ist/sind.

**19.** Diagnostische Zusammensetzung zur Diagnose von chronischen entzündlichen Atemwegserkrankungen, die gleichzeitig alpha-Enolaseprotein und Cytokeratin 18-Protein umfasst.

**20.** Zusammensetzung gemäss Anspruch 19, wobei das alpha-Enolaseprotein von Säugern, ausgewählt aus der Gruppe bestehend aus Mensch, Maus, Ratte, Kaninchen, Schwein, Kuh und Ziege, stammt.

**21.** Zusammensetzung gemäss Anspruch 20, wobei das alpha-Enolaseprotein die Aminosäuresequenz SEQ ID NO: 1 aufweist.

**22.** Zusammensetzung gemäss Anspruch 20, wobei das alpha-Enolaseprotein die Aminosäuresequenz SEQ ID NO: 2 aufweist.

**23.** Zusammensetzung gemäss Anspruch 19, wobei das Cytokeratin 18-Protein von Säugern, ausgewählt aus der Gruppe bestehend aus Mensch, Maus, Ratte, Kaninchen, Schwein, Kuh und Ziege, stammt.

**24.** Zusammensetzung gemäss Anspruch 23, wobei das alpha-Enolaseprotein die Aminosäuresequenz SEQ ID NO: 4 aufweist.

**25.** Zusammensetzung gemäss Anspruch 19, wobei eine der chronischen entzündlichen Atemwegserkrankungen Bronchialasthma oder chronisch obstruktive Lungenerkrankung ist.

**26.** Zusammensetzung gemäss Anspruch 25, wobei das Bronchialasthma schweres Asthma oder Asthma aufgrund von Aspirin-Überempfindlichkeit ist.

**27.** Zusammensetzung zur Detektion von Autoantikörpern, die gleichzeitig alpha-Enolaseprotein und Cytokeratin 18-Protein umfasst.

**28.** Zusammensetzung gemäss Anspruch 27, wobei das alpha-Enolaseprotein von Säugern, ausgewählt aus der Gruppe bestehend aus Mensch, Maus, Ratte, Kaninchen, Schwein, Kuh und Ziege, stammt.

**29.** Zusammensetzung gemäss Anspruch 28, wobei das alpha-Enolaseprotein die Aminosäuresequenz SEQ ID NO: 1 aufweist.

**30.** Zusammensetzung gemäss Anspruch 28, wobei das alpha-Enolaseprotein die Aminosäuresequenz SEQ ID NO: 2 aufweist.

**31.** Zusammensetzung gemäss Anspruch 27, wobei das Cytokeratin 18-Protein von Säugern, ausgewählt aus der Gruppe bestehend aus Mensch, Maus, Ratte, Kaninchen, Schwein, Kuh und Ziege, stammt.

**32.** Zusammensetzung gemäss Anspruch 31, wobei das alpha-Enolaseprotein die Aminosäuresequenz SEQ ID NO: 4 aufweist.

**33.** Verfahren zur Detektion von Autoantikörpern durch Verwendung von Zusammensetzungen zur Detektion von Autoantikörpern, die alpha-Enolaseprotein und Cytokeratin 18-Protein enthalten, umfassend die Schritte:

(a) Gewinnen biologischer Proben von einem Subjekt;
(b) In-Kontakt-Bringen der biologischen Proben mit irgendeiner der Zusammensetzungen gemäss den Ansprüchen 27 bis 32, um die Bildung eines Immunkomplexes zu induzieren, und
(c) Detektieren der Bildung des Immunkomplexes in dem Subjekt, zur Bestimmung der Gegenwart von Autoantikörpern gegen alpha-Enolaseprotein und Cytokeratin 18-Proteine.

**34.** Verfahren gemäss Anspruch 33, wobei das alpha-Enolaseprotein von Säugern, ausgewählt aus der Gruppe bestehend aus Mensch, Maus, Ratte, Kaninchen, Schwein, Kuh und Ziege, stammt.

**35.** Verfahren gemäss Anspruch 34, wobei das alpha-Enolaseprotein die Aminosäuresequenz SEQ ID NO: 1 aufweist.

**36.** Verfahren gemäss Anspruch 34, wobei das alpha-Enolaseprotein die Aminosäuresequenz SEQ ID NO: 2 aufweist.

**37.** Verfahren gemäss Anspruch 33, wobei das Cytokeratin 18-Protein von Säugern, ausgewählt aus der Gruppe bestehend aus Mensch, Maus, Ratte, Kaninchen, Schwein, Kuh und Ziege, stammt.

**38.** Verfahren gemäss Anspruch 37, wobei das Cytokeratin 18-Protein die Aminosäuresequenz SEQ ID NO: 4 aufweist.

**39.** Verfahren gemäss Anspruch 33, wobei das Subjekt ein Patient mit chronischen entzündlichen Erkrankungen ist.

**40.** Diagnostische Zusammensetzung, umfassend alpha-Enolaseprotein, zur Verwendung in einem Verfahren zur Diagnose von chronischen entzündlichen Atemwegserkrankungen, einschliesslich Bronchialasthma und chronisch obstruktiver Lungenerkrankung, durch Verwendung einer diagnostischen Zusammensetzung, die alpha-Enolaseprotein enthält, umfassend die Schritte:

(a) Auswählen eines Subjekts mit Verdacht auf chronische entzündliche Atemwegserkrankungen;
(b) intradermale Injektion einer diagnostischen Zusammensetzung zur Diagnose von chronischen entzündlichen Atemwegserkrankungen, umfassend alpha-Enolaseprotein, an das Subjekt; und
(c) Diagnose von chronischen entzündlichen Atemwegserkrankungen durch Untersuchen des injizierten Hautbereichs 24 bis 72 Stunden nach der Injektion, um das Vorhandensein von Hautschwellungen zu erkennen, und messen der Grösse der Hautschwellungen, um die Gegenwart einer verzögerten überempfindlichen Reaktion oder einer späten Reaktion der Haut auf alpha-Enolaseprotein zu bestimmen.

**41.** Diagnostische Zusammensetzung zur Verwendung gemäss Anspruch 40, wobei das alpha-Enolaseprotein von Säugern, ausgewählt aus der Gruppe bestehend aus Mensch, Maus, Ratte, Kaninchen, Schwein, Kuh und Ziege, stammt.

**42.** Diagnostische Zusammensetzung zur Verwendung gemäss Anspruch 41, wobei das alpha-Enolaseprotein die Aminosäuresequenz SEQ ID NO: 1 aufweist.

**43.** Diagnostische Zusammensetzung zur Verwendung gemäss Anspruch 41, wobei das alpha-Enolaseprotein die Aminosäuresequenz SEQ ID NO: 2 aufweist.

**44.** Diagnostische Zusammensetzung zur Verwendung gemäss Anspruch 40, wobei eine der chronischen entzündlichen Atemwegserkrankungen Bronchialasthma oder chronisch obstruktive Lungenerkrankung ist.

**45.** Diagnostische Zusammensetzung zur Verwendung gemäss Anspruch 44, wobei das Bronchialasthma schweres Asthma oder Asthma aufgrund von Aspirin-Überempfindlichkeit ist.

**Revendications**

**1.** Composition pharmaceutique pour une utilisation dans la prévention ou le traitement des maladies inflammatoires chroniques des voies respiratoires comprenant une protéine alpha-énolase comme principe actif.

**2.** Composition de la revendication 1 pour l'utilisation selon la revendication 1, dans laquelle la protéine alpha-énolase provient de mammifères choisis dans le groupe constitué d'un être humain, d'une souris, d'un rat, d'un lapin, d'un cochon, d'une vache et d'une chèvre.

**3.** Composition de la revendication 2 pour l'utilisation selon la revendication 2, dans laquelle la protéine alpha-énolase a une séquence d'acides aminés de la séquence SEQ ID NO : 1.

**4.** Composition de la revendication 2 pour l'utilisation selon la revendication 2, dans laquelle la protéine alpha-énolase a une séquence d'acides aminés de la séquence SEQ ID NO : 2.

**5.** Composition de la revendication 1 pour l'utilisation selon la revendication 1, dans laquelle l'une des maladies inflammatoires chroniques des voies respiratoires est l'asthme bronchique ou la bronchopneumopathie chronique obstructive.

**6.** Composition de la revendication 5 pour l'utilisation selon la revendication 5, dans laquelle l'asthme bronchique est un asthme sévère ou un asthme hypersensible à l'aspirine.

7. Procédé de diagnostic de maladies inflammatoires chroniques des voies respiratoires en utilisant des compositions de diagnostic contenant une protéine alpha-énolase comme principe actif, comprenant les étapes qui consistent :

   (a) à obtenir des échantillons biologiques d'un sujet suspecté d'être atteint de maladies inflammatoires chroniques des voies respiratoires telles que l'asthme bronchique ou la bronchopneumopathie chronique obstructive ;
   (b) à mettre en contact les échantillons biologiques avec une composition de diagnostic destinée au diagnostic de maladies inflammatoires chroniques des voies respiratoires comprenant une protéine alpha-énolase pour induire la formation d'un complexe immun ; et
   (c) à diagnostiquer lesdites maladies inflammatoires chroniques des voies respiratoires chez le sujet en détectant le complexe immun pour déterminer la présence d'auto-anticorps contre la protéine alpha-énolase.

8. Procédé de la revendication 7, dans lequel la protéine alpha-énolase provient de mammifères choisis dans le groupe constitué d'un être humain, d'une souris, d'un rat, d'un lapin, d'un cochon, d'une vache et d'une chèvre.

9. Procédé de la revendication 8, dans lequel la protéine alpha-énolase a la séquence d'acides aminés de la séquence SEQ ID NO : 1.

10. Procédé de la revendication 8, dans lequel la protéine alpha-énolase a la séquence d'acides aminés de la séquence SEQ ID NO : 2.

11. Procédé de la revendication 7, dans lequel l'une des maladies inflammatoires chroniques des voies respiratoires est l'asthme bronchique ou la bronchopneumopathie chronique obstructive.

12. Procédé de la revendication 11, dans lequel l'asthme bronchique est un asthme sévère ou un asthme hypersensible à l'aspirine.

13. Procédé de criblage d'un agent thérapeutique pour des maladies inflammatoires chroniques des voies respiratoires en utilisant une composition destinée au criblage d'un agent thérapeutique pour des maladies inflammatoires chroniques des voies respiratoires, comprenant un ou plusieurs élément(s) parmi une protéine alpha-énolase, des anticorps contre ladite protéine alpha-énolase, ou des auto-anticorps contre l'alpha-énolase obtenu(s) à partir de patients atteints desdites maladies inflammatoires chroniques des voies respiratoires, comprenant les étapes qui consistent :

   (a) à isoler des auto-anticorps à partir d'échantillons biologiques obtenus à partir de patients atteints de maladies inflammatoires chroniques des voies respiratoires telles que l'asthme bronchique ou la bronchopneumopathie chronique obstructive ;
   (b) à mettre en contact des matières d'essai avec les auto-anticorps obtenus dans l'étape (a) ci-dessus ; et
   (c) à sélectionner un agent thérapeutique pour des maladies inflammatoires chroniques des voies respiratoires en déterminant si les matières d'essai peuvent inhiber la liaison des auto-anticorps à la protéine alpha-énolase, les effets cytotoxiques des auto-anticorps sur des cellules exprimant la protéine alpha-énolase ou la sécrétion de médiateurs inflammatoires y compris la cytokine inflammatoire par des cellules exprimant la protéine alpha-énolase induite par les auto-anticorps.

14. Procédé de criblage selon la revendication 13, dans lequel la protéine alpha-énolase provient de mammifères choisis dans le groupe constitué d'un être humain, d'une souris, d'un rat, d'un lapin, d'un cochon, d'une vache et d'une chèvre.

15. Procédé de criblage selon la revendication 14, dans lequel la protéine alpha-énolase a la séquence d'acides aminés de la séquence SEQ ID NO : 1.

16. Procédé de criblage selon la revendication 14, dans lequel la protéine alpha-énolase a la séquence d'acides aminés de la séquence SEQ ID NO : 2.

17. Procédé de criblage selon la revendication 13, dans lequel l'une des maladies inflammatoires chroniques des voies respiratoires est l'asthme bronchique ou la bronchopneumopathie chronique obstructive.

18. Procédé de l'une quelconque des revendications 13 à 17, dans lequel lesdits échantillons biologiques sont une ou plusieurs matière(s) choisie(s) dans le groupe constitué par le sang, le plasma, le sérum, l'urine, les larmes, la salive, le crachat, la sécrétion nasale, la sécrétion bronchique, le fluide de lavage bronchique, la sécrétion pulmonaire et le fluide de lavage alvéolaire.

**19.** Composition de diagnostic destinée au diagnostic de maladies inflammatoires chroniques des voies respiratoires comprenant une protéine alpha-énolase et une protéine cytokératine 18 simultanément.

**20.** Composition de la revendication 19, dans laquelle la protéine alpha-énolase provient de mammifères choisis dans le groupe constitué d'un être humain, d'une souris, d'un rat, d'un lapin, d'un cochon, d'une vache et d'une chèvre.

**21.** Composition de la revendication 20, dans laquelle la protéine alpha-énolase a la séquence d'acides aminés de la séquence SEQ ID NO : 1.

**22.** Composition de la revendication 20, dans laquelle la protéine alpha-énolase a la séquence d'acides aminés de la séquence SEQ ID NO : 2.

**23.** Composition de la revendication 19, dans laquelle la protéine cytokératine 18 provient de mammifères choisis dans le groupe constitué d'un être humain, d'une souris, d'un rat, d'un lapin, d'un cochon, d'une vache et d'une chèvre.

**24.** Composition de la revendication 23, dans laquelle la protéine alpha-énolase a la séquence d'acides aminés de la séquence SEQ ID NO : 4.

**25.** Composition de la revendication 19, dans laquelle l'une des maladies inflammatoires chroniques des voies respiratoires est l'asthme bronchique ou la bronchopneumopathie chronique obstructive.

**26.** Composition de la revendication 25, dans laquelle l'asthme bronchique est un asthme sévère ou un asthme hypersensible à l'aspirine.

**27.** Composition pour détecter des auto-anticorps comprenant une protéine alpha-énolase et une protéine cytokératine 18 simultanément.

**28.** Composition de la revendication 27, dans laquelle la protéine alpha-énolase provient de mammifères choisis dans le groupe constitué d'un être humain, d'une souris, d'un rat, d'un lapin, d'un cochon, d'une vache et d'une chèvre.

**29.** Composition de la revendication 28, dans laquelle la protéine alpha-énolase a la séquence d'acides aminés de la séquence SEQ ID NO : 1.

**30.** Composition de la revendication 28, dans laquelle la protéine alpha-énolase a la séquence d'acides aminés de la séquence SEQ ID NO : 2.

**31.** Composition de la revendication 27, dans laquelle la protéine cytokératine 18 provient de mammifères choisis dans le groupe constitué d'un être humain, d'une souris, d'un rat, d'un lapin, d'un cochon, d'une vache et d'une chèvre.

**32.** Composition de la revendication 31, dans laquelle la protéine alpha-énolase a la séquence d'acides aminés de la séquence SEQ ID NO : 4.

**33.** Procédé de détection d'auto-anticorps en utilisant des compositions de détection d'auto-anticorps contenant une protéine alpha-énolase et une protéine cytokératine 18, comprenant les étapes qui consistent :

(a) à obtenir des échantillons biologiques d'un suj et ;
(b) à mettre en contact les échantillons biologiques avec l'une quelconque des compositions des revendications 27 à 32 pour induire la formation d'un complexe immun ; et
(c) à détecter la formation dudit complexe immun chez le sujet pour déterminer la présence d'auto-anticorps contre la protéine alpha-énolase et les protéines cytokératine 18.

**34.** Procédé de la revendication 33, dans lequel la protéine alpha-énolase provient de mammifères choisis dans le groupe constitué d'un être humain, d'une souris, d'un rat, d'un lapin, d'un cochon, d'une vache et d'une chèvre.

**35.** Procédé de la revendication 34, dans lequel la protéine alpha-énolase a la séquence d'acides aminés de la séquence SEQ ID NO : 1.

**36.** Procédé de la revendication 34, dans lequel la protéine alpha-énolase a la séquence d'acides aminés de la séquence SEQ ID NO : 2.

**37.** Procédé de la revendication 33, dans lequel la protéine cytokératine 18 provient de mammifères choisis dans le groupe constitué d'un être humain, d'une souris, d'un rat, d'un lapin, d'un cochon, d'une vache et d'une chèvre.

**38.** Procédé de la revendication 37, dans lequel la protéine cytokératine 18 a la séquence d'acides aminés de la séquence SEQ ID NO : 4.

**39.** Procédé de la revendication 33, dans lequel le sujet est un patient atteint de maladies inflammatoires chroniques.

**40.** Composition de diagnostic comprenant une protéine alpha-énolase pour une utilisation dans un procédé pour le diagnostic de maladies inflammatoires chroniques des voies respiratoires y compris l'asthme bronchique et la bronchopneumopathie chronique obstructive en utilisant une composition de diagnostic contenant une protéine alpha-énolase, comprenant les étapes qui consistent :

(a) à sélectionner un sujet suspecté d'être atteint de maladies inflammatoires chroniques des voies respiratoires ;
(b) à injecter par voie intradermique au sujet une composition de diagnostic destinée au diagnostic de maladies inflammatoires chroniques des voies respiratoires comprenant une protéine alpha-énolase ; et
(c) à diagnostiquer des maladies inflammatoires chroniques des voies respiratoires en examinant la partie de la peau injectée après 24 à 72 heures de l'injection pour détecter la présence d'un gonflement cutané et pour mesurer la taille du gonflement cutané afin de déterminer la présence d'une réaction d'hypersensibilité de type retardée ou d'une réaction cutanée tardive avec la protéine alpha-énolase.

**41.** Composition de diagnostic pour une utilisation selon la revendication 40, dans laquelle la protéine alpha-énolase provient de mammifères choisis dans le groupe constitué d'un être humain, d'une souris, d'un rat, d'un lapin, d'un cochon, d'une vache et d'une chèvre.

**42.** Composition de diagnostic pour une utilisation selon la revendication 41, dans laquelle la protéine alpha-énolase a la séquence d'acides aminés de la séquence SEQ ID NO : 1.

**43.** Composition de diagnostic pour une utilisation selon la revendication 41, dans laquelle la protéine alpha-énolase a la séquence d'acides aminés de la séquence SEQ ID NO : 2.

**44.** Composition de diagnostic pour une utilisation selon la revendication 40, dans laquelle l'une des maladies inflammatoires chroniques des voies respiratoires est l'asthme bronchique ou la bronchopneumopathie chronique obstructive.

**45.** Composition de diagnostic pour une utilisation selon la revendication 44, dans laquelle l'asthme bronchique est un asthme sévère ou un asthme hypersensible à l'aspirine.

Fig. 1

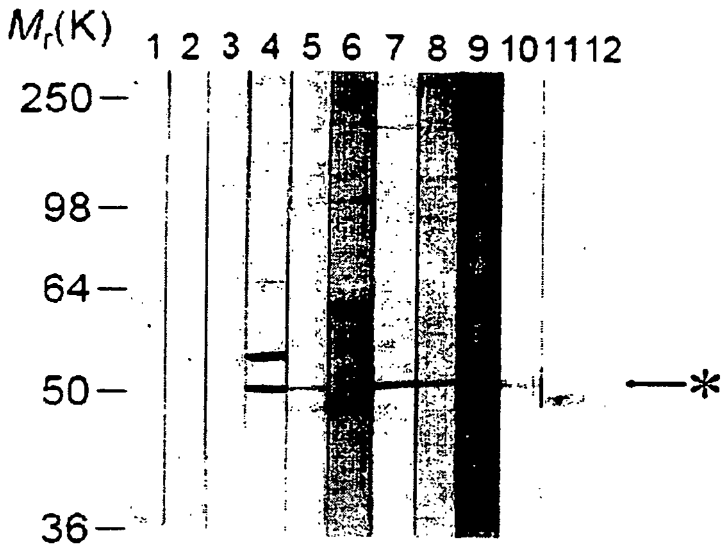


Fig. 2

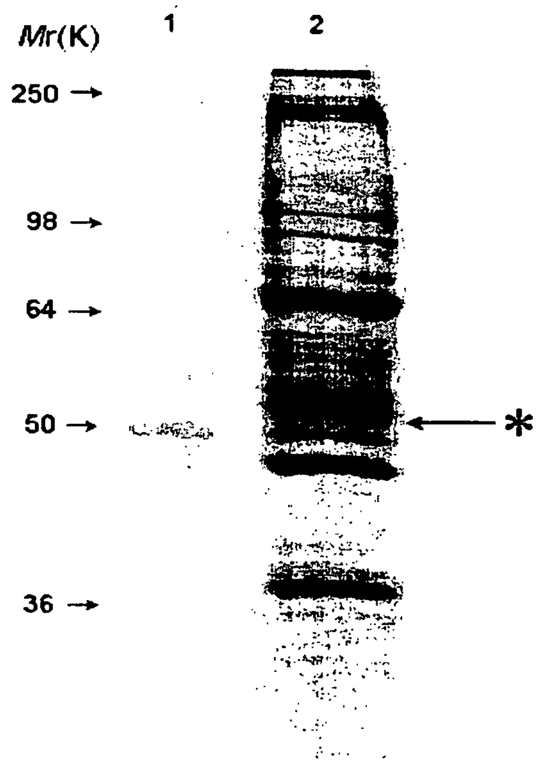

Fig. 3

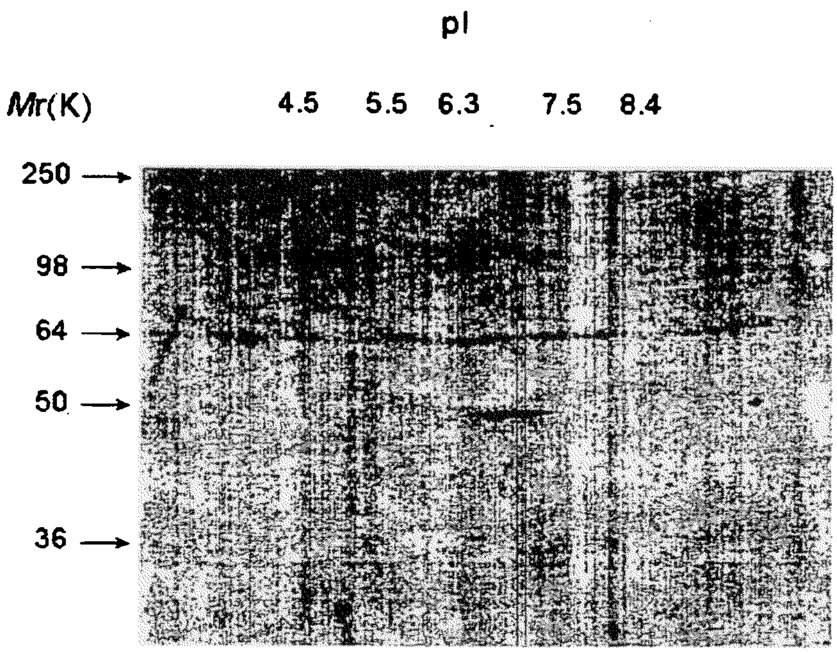
pI
Mr(K)
4.5
5.5
6.3
7.5
8.4
250
98
64
50
36

Fig. 4

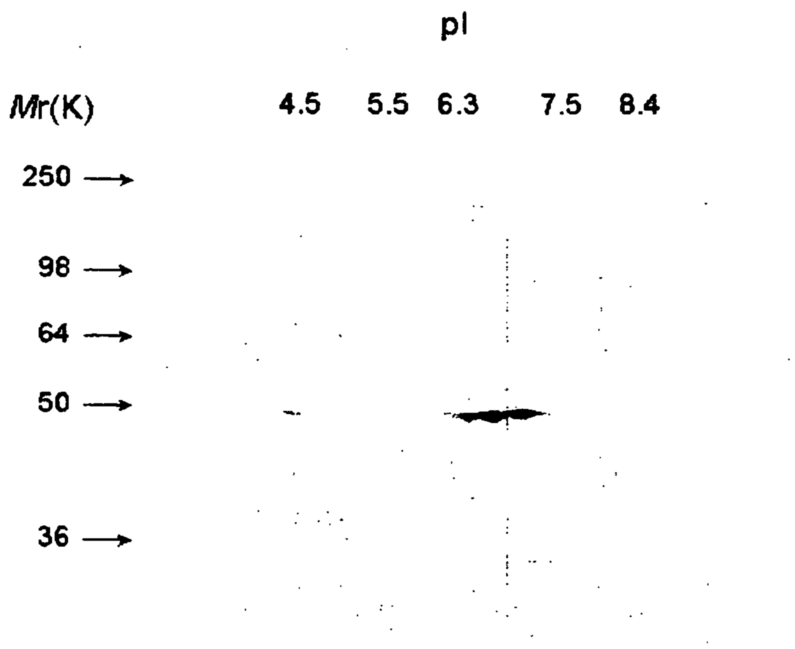
pI
Mr(K)
4.5
5.5
6.3
7.5
8.4
250
98
64
50
36

Fig. 5

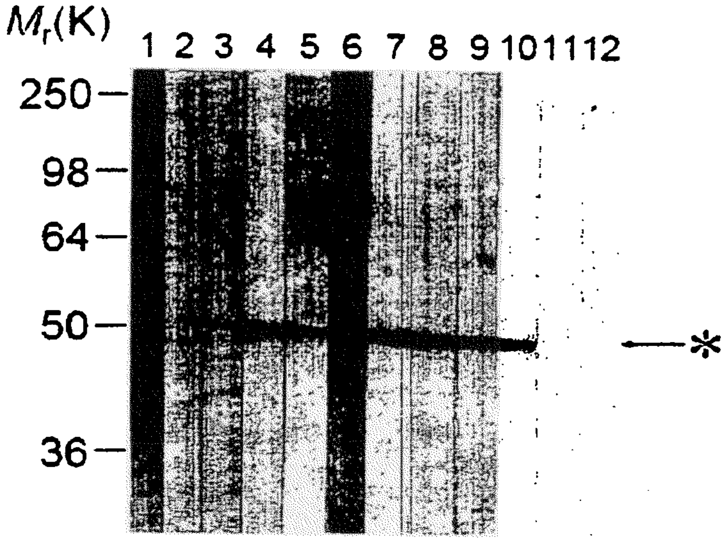
$M_r$(K)
1 2 3 4 5 6 7 8 9 10 11 12
250
98
64
50
36
*

Fig. 6

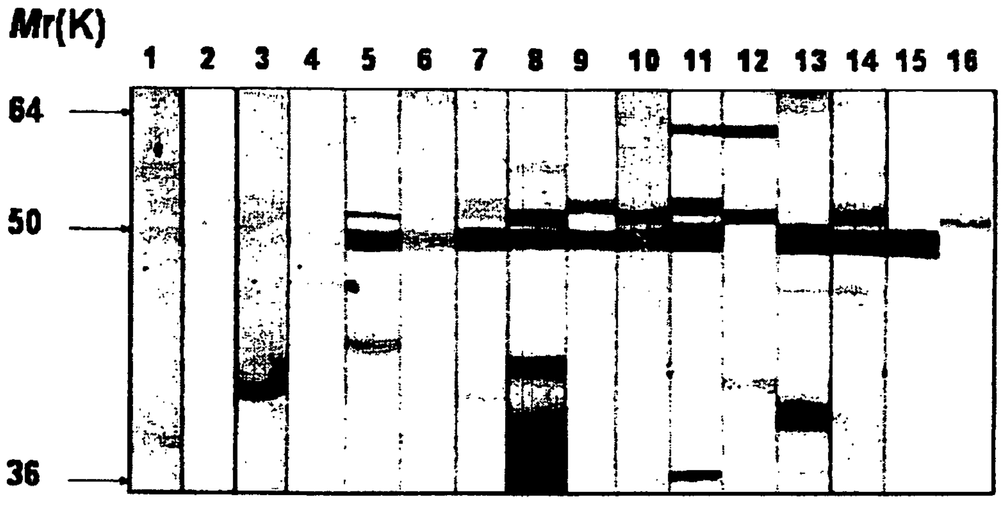
Mr(K)
1 2 3 4 5 6 7 8 9 10 11 12 13 14 15 16
64
50
36

Fig. 7

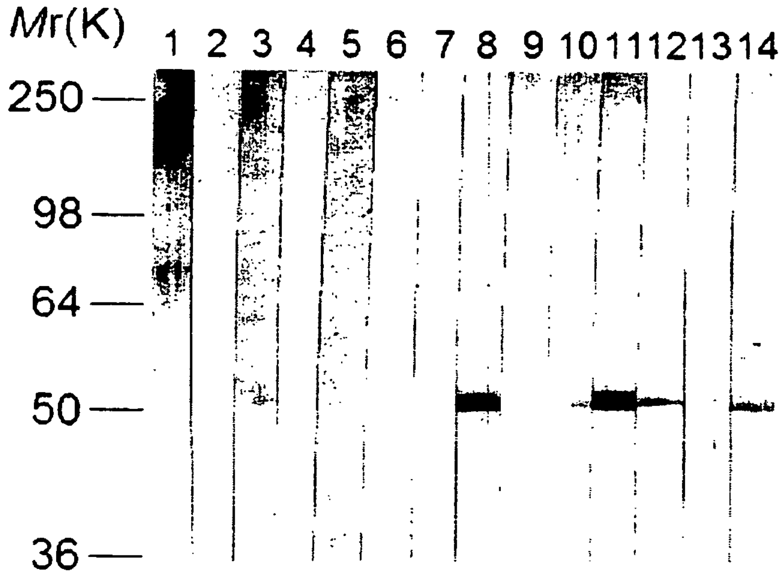
Mr(K)
1 2 3 4 5 6 7 8 9 10 11 12 13 14
250
98
64
50
36

Fig. 8

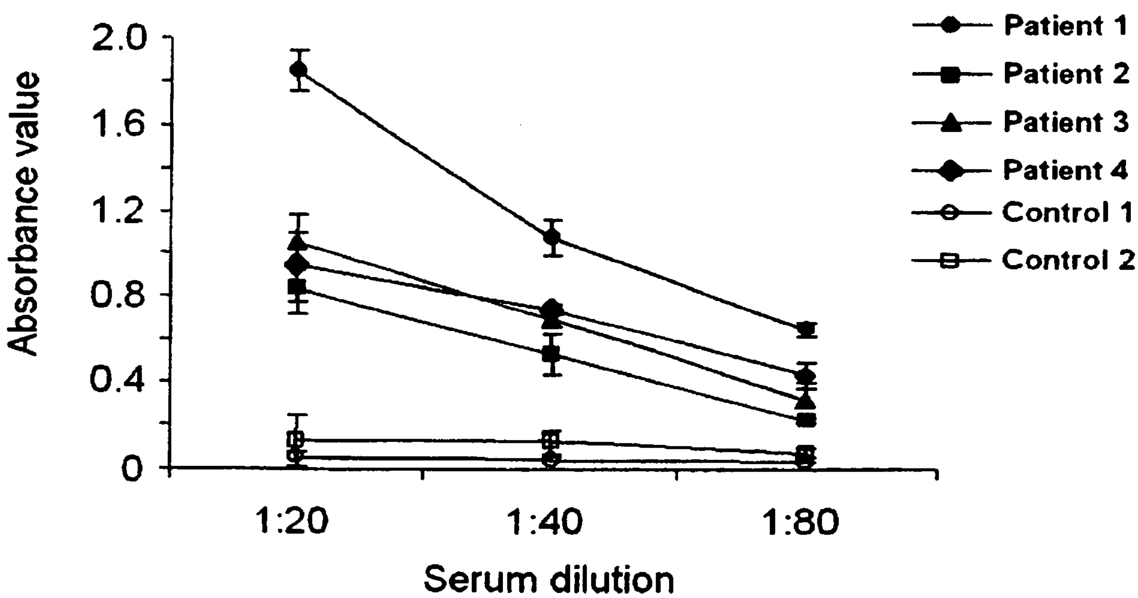
Absorbance value
2.0
1.6
1.2
0.8
0.4
0
1:20
1:40
1:80
Serum dilution
Patient 1
Patient 2
Patient 3
Patient 4
Control 1
Control 2

Fig. 9

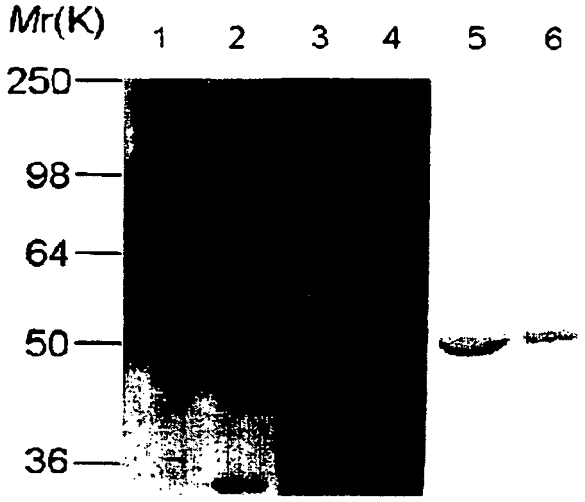
Mr(K)
1 2 3 4 5 6
250
98
64
50
36

Fig. 10a

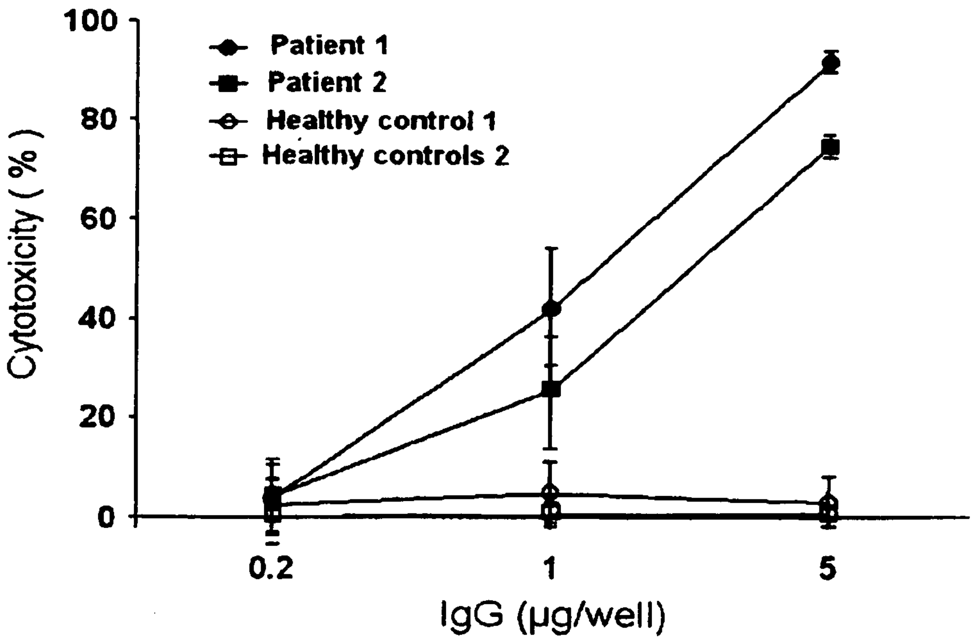
Patient 1
Patient 2
Healthy control 1
Healthy controls 2
Cytotoxicity ( % )
100
80
60
40
20
0
0.2
1
5
IgG (μg/well)

Fig. 10b

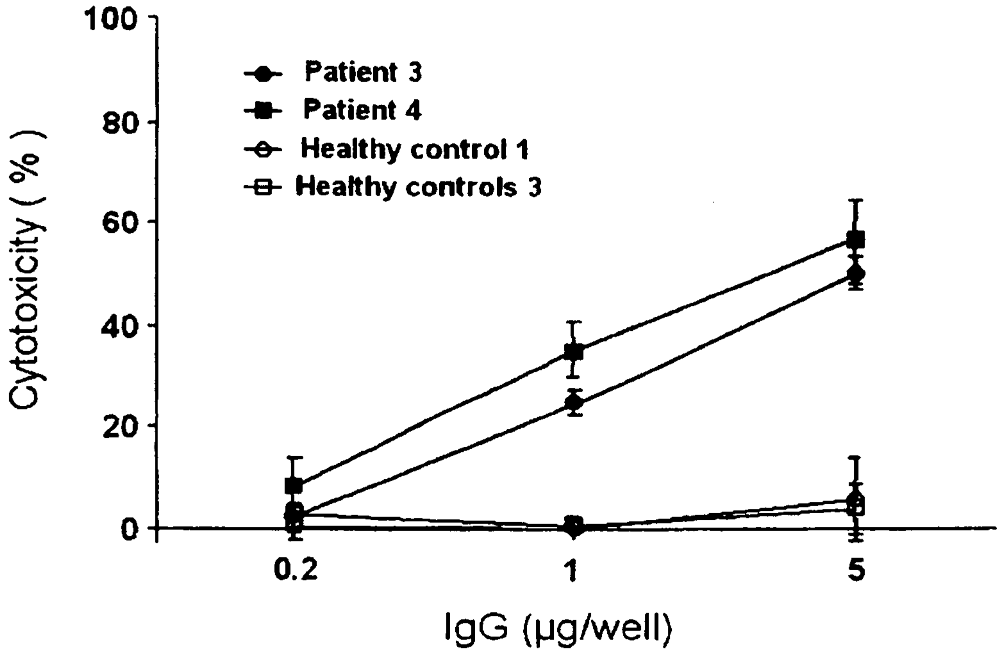


Fig. 10c

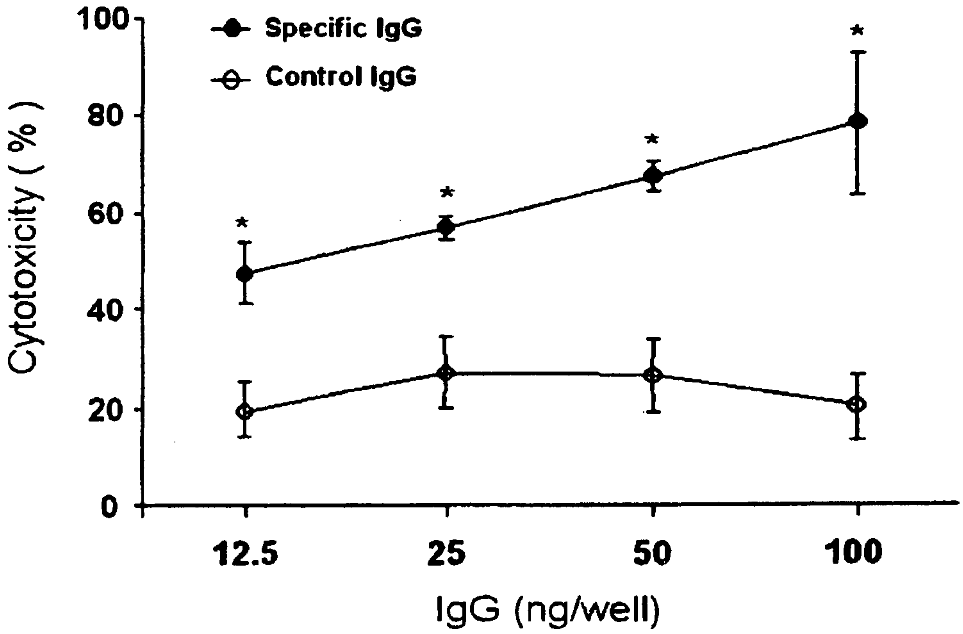

Fig. 11

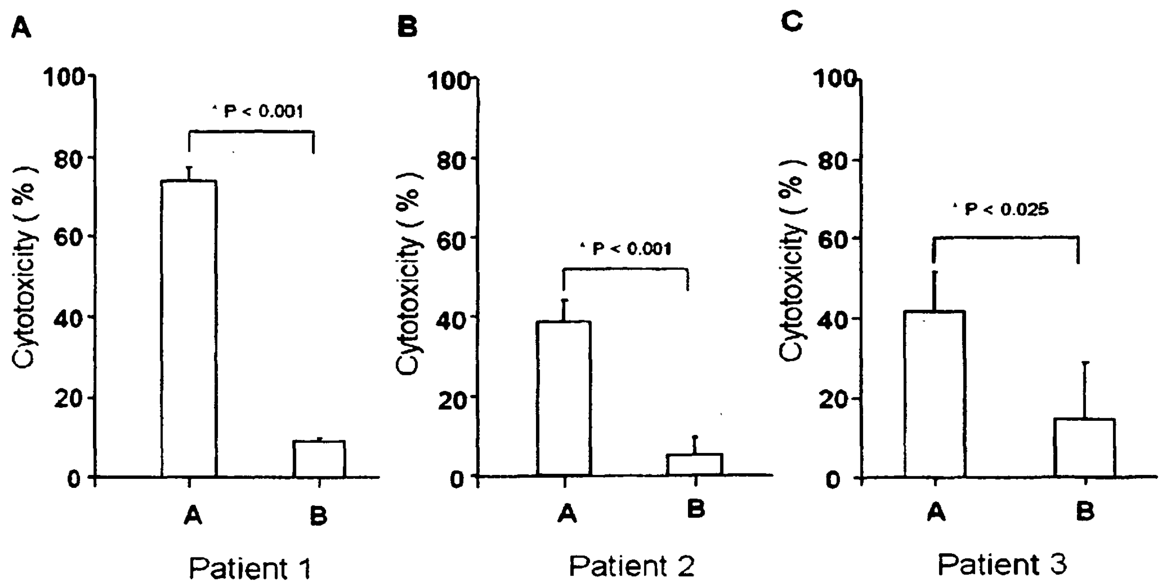


Fig. 12a

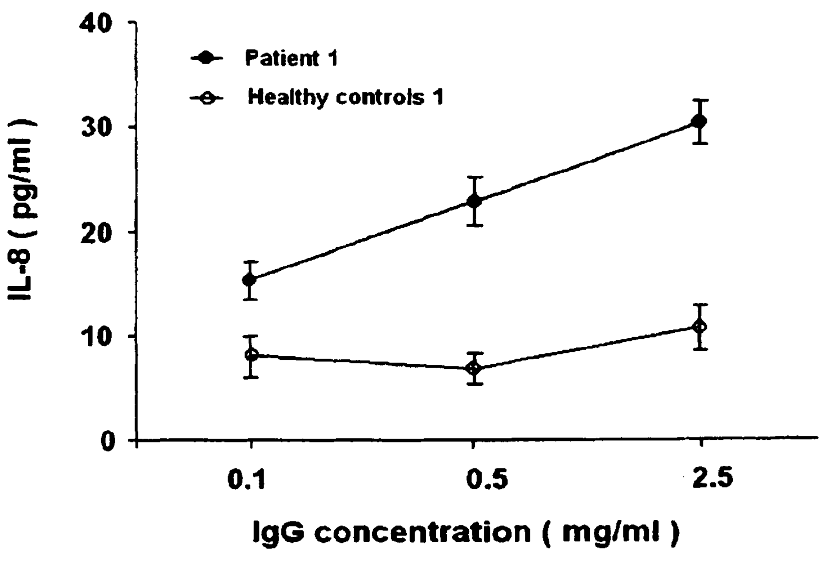

Fig. 12b

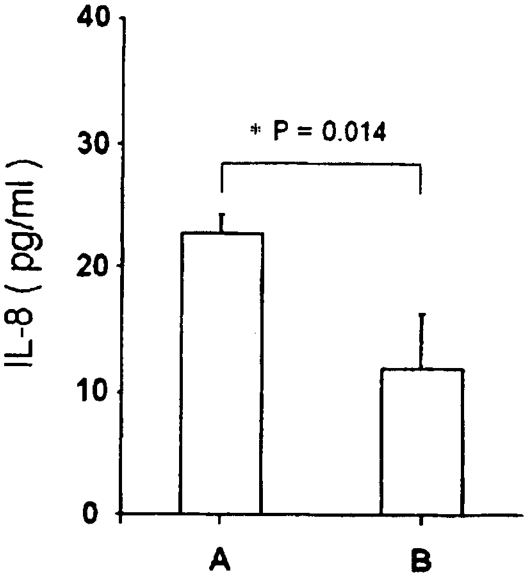

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005032572 A2
- US 5874080 A
- KR 1020050000098
- KR 1020050013584
- KR 102005135154


### Non-patent literature cited in the description


- **WARDLAW A et al.** *Clin Exp Allergy,* 2005, vol. 35, 1254-62
- **GUERRA S.** *Curr Opin Pulm Med,* 2005, vol. 11, 7-13
- Global Initiative for Asthma. NIH Publication No. 02-3659, 2002
- **LEMANSKE, R.F., JR et al.** *JAMA,* 1997, vol. 278, 1855-1873
- **PEARCE, N. et al.** *Thorax,* 1999, vol. 54, 268-272
- Global Initiative for Asthma. NIH Publication No. 02-3659, 2002, 50-66
- **MONTEFORT, S. et al.** *Clin Exp Allergy,* 1992, vol. 22, 511-520
- **CHANEZ P.** *Eur Respir J,* 2005, vol. 25, 945-6
- **KAROL SA et al.** *Vrach Delo,* 1966, vol. 4, 28-32
- **CALLERAME ML et al.** *N Eng J Med,* 1971, vol. 284, 459-64
- **MOLINA, C. et al.** *Clin Allergy,* 1977, vol. 7, 137-45
- **ROTTEM M ; SHOENFELD Y.** *Int Arch Allergy Immunol,* 2003, vol. 132, 210-4
- GOLD workshop summary. *Am J Respir Crit Care Med,* 2001, vol. 163, 1256-1276
- **CELLI BR et al.** *Eur Respir J,* 2004, vol. 23, 932-46
- **GUERRA S.** *Curr Opin Pulm Med,* 2005, vol. 11, 17-13
- **O'BYRNE PM ; POSTMA DS.** *Am J Respir Crit Care Med,* 1999, vol. 159, S41-S66
- **HODGE S et al.** *Eur Respir J,* 2005, vol. 25, 447-54
- *Am J Respir Crit Care Med,* 2001, vol. 163, 1256-1276
- **WAGNER V et al.** *Acta Allergol,* 1965, vol. 20, 1-9
- **AGUSTI A et al.** *Thorax,* 2003, vol. 58, 832-834
- **TARASEVICIENE-STEWART L et al.** *Am J Respir Crit Care Med.,* 2005, vol. 171, 734-42
- **PANCHOLI V.** *Cell Mol Life Sci.,* 2001, vol. 58, 902-920
- **GIALLONGO A et al.** *Proc. Natl. Acad. Sci. U.S.A.,* 1986, vol. 83, 6741-6745
- Remington's Pharmaceutical Science. Mack Publishing Company
- **BEALES PE et al.** *Autoimmunity,* 2000, vol. 32, 109-113
- **NAHM DH.** *Am J Respir Crit Care Med,* 2002, vol. 165, 1536-9
- **SAMBROOK et al.** Molecular Cloning, A Laboratory Manual. Cold Spring Harbor laboratory Press, 2001
- **PARK HS.** *Clin Exp Allergy,* 1995, vol. 25, 28-40
- **GIARD et al.** *J Natl Cancer Inst,* 1973, vol. 51, 1417-23
- **LEE KH et al.** *Arthritis Rheum.,* 2003, vol. 48, 2025-2035
- **DARLINGTON GJ et al.** *J Nat'l Cancer Inst,* 1980, vol. 64, 809-819
- **MARTIN S et al.** *Tissue Antigens,* 1991, vol. 37, 152-155
- **LEBING W et al.** *Vox Sang,* 2003, vol. 84, 193-201
- **XIU Q et al.** *Clin Exp Allergy,* 1995, vol. 25, 51-9
- **MAHLER DA.** *Chest,* 2004, vol. 126, 926-934

## SEQUENCE LISTING

<110> JEON. SOOK YEONG NAHM. DONG HO

<120> Composition for prevention, treatment and diagnosis of chronic inflammatory airway diseases

<130> 05pp489

<150> KR 10-2005-0000098
<151> 2005-01-03

<150> KR 10-2005-0013584
<151> 2005-02-18

<150> KR 10-2005-135154
<151> 2005-12-30

<160> 5

<170> Kopatent In 1.71

<210> 1
<211> 434
<212> PRT
<213> Homo sapiens

<220>
<221> CHAIN
<222> (1) .. (434)

<400> 1

```
Met Ser Ile Leu Lys Ile His Ala Arg Glu Ile Phe Asp Ser Arg Gly
1               5                   10                  15

Asn Pro Thr Val Glu Val Asp Leu Phe Thr Ser Lys Gly Leu Phe Arg
            20                  25                  30

Ala Ala Val Pro Ser Gly Ala Ser Thr Gly Ile Tyr Glu Ala Leu Glu
        35                  40                  45

Leu Arg Asp Asn Asp Lys Thr Arg Tyr Met Gly Lys Gly Val Ser Lys
    50                  55                  60
```

Ala Val Glu His Ile Asn Lys Thr Ile Ala Pro Ala Leu Val Ser Lys
65 70 75 80

Lys Leu Asn Val Thr Glu Gln Glu Lys Ile Asp Lys Leu Met Ile Glu
85 90 95

Met Asp Gly Thr Glu Asn Lys Ser Lys Phe Gly Ala Asn Ala Ile Leu
100 105 110

Gly Val Ser Leu Ala Val Cys Lys Ala Gly Ala Val Glu Lys Gly Val
115 120 125

Pro Leu Tyr Arg His Ile Ala Asp Leu Ala Gly Asn Ser Glu Val Ile
130 135 140

Leu Pro Val Pro Ala Phe Asn Val Ile Asn Gly Gly Ser His Ala Gly
145 150 155 160

Asn Lys Leu Ala Met Gln Glu Phe Met Ile Leu Pro Val Gly Ala Ala
165 170 175

Asn Phe Arg Glu Ala Met Arg Ile Gly Ala Glu Val Tyr His Asn Leu
180 185 190

Lys Asn Val Ile Lys Glu Lys Tyr Gly Lys Asp Ala Thr Asn Val Gly
195 200 205

Asp Glu Gly Gly Phe Ala Pro Asn Ile Leu Glu Asn Lys Glu Gly Leu
210 215 220

Glu Leu Leu Lys Thr Ala Ile Gly Lys Ala Gly Tyr Thr Asp Lys Val
225 230 235 240

Val Ile Gly Met Asp Val Ala Ala Ser Glu Phe Phe Arg Ser Gly Lys
245 250 255

Tyr Asp Leu Asp Phe Lys Ser Pro Asp Asp Pro Ser Arg Tyr Ile Ser
260 265 270

Pro Asp Gln Leu Ala Asp Leu Tyr Lys Ser Phe Ile Lys Asp Tyr Pro
275 280 285

Val Val Ser Ile Glu Asp Pro Phe Asp Gln Asp Asp Trp Gly Ala Trp
290 295 300

Gln Lys Phe Thr Ala Ser Ala Gly Ile Gln Val Val Gly Asp Asp Leu
305 310 315 320

Thr Val Thr Asn Pro Lys Arg Ile Ala Lys Ala Val Asn Glu Lys Ser
325 330 335

Cys Asn Cys Leu Leu Leu Lys Val Asn Gln Ile Gly Ser Val Thr Glu
340 345 350

Ser Leu Gln Ala Cys Lys Leu Ala Gln Ala Asn Gly Trp Gly Val Met
355 360 365

Val Ser His Arg Ser Gly Glu Thr Glu Asp Thr Phe Ile Ala Asp Leu
370 375 380

Val Val Gly Leu Cys Thr Gly Gln Ile Lys Thr Gly Ala Pro Cys Arg
385 390 395 400

Ser Glu Arg Leu Ala Lys Tyr Asn Gln Leu Leu Arg Ile Glu Glu Glu
405 410 415

Leu Gly Ser Lys Ala Lys Phe Ala Gly Arg Asn Phe Arg Asn Pro Leu
420 425 430

Ala Lys

<210> 2
<211> 434
<212> PRT
<213> Mus musculus

<220>
<221> CHAIN
<222> (1) .. (434)

<400> 2

Met Ser Ile Leu Arg Ile His Ala Arg Glu Ile Phe Asp Ser Arg Gly
1 5 10 15

Asn Pro Thr Val Glu Val Asp Leu Tyr Thr Ala Lys Gly Leu Phe Arg
20 25 30

Ala Ala Val Pro Ser Gly Ala Ser Thr Gly Ile Tyr Glu Ala Leu Glu
35 40 45

Leu Arg Asp Asn Asp Lys Thr Arg Phe Met Gly Lys Gly Val Ser Gln
50 55 60

Ala Val Glu His Ile Asn Lys Thr Ile Ala Pro Ala Leu Val Ser Lys
65 70 75 80

Lys Val Asn Val Val Glu Gln Glu Lys Ile Asp Lys Leu Met Ile Glu
85 90 95

Met Asp Gly Thr Glu Asn Lys Ser Lys Phe Gly Ala Asn Ala Ile Leu
100 105 110

Gly Val Ser Leu Ala Val Cys Lys Ala Gly Ala Val Glu Lys Gly Val
115 120 125

Pro Leu Tyr Arg His Ile Ala Asp Leu Ala Gly Asn Pro Glu Val Ile
130 135 140

Leu Pro Val Pro Ala Phe Asn Val Ile Asn Gly Gly Ser His Ala Gly
145 150 155 160

Asn Lys Leu Ala Met Gln Glu Phe Met Ile Leu Pro Val Gly Ala Ser
165 170 175

Ser Phe Arg Glu Ala Met Arg Ile Gly Ala Glu Val Tyr His Asn Leu
180 185 190

Lys Asn Val Ile Lys Glu Lys Tyr Gly Lys Asp Ala Thr Asn Val Gly
195 200 205

Asp Glu Gly Gly Phe Ala Pro Asn Ile Leu Glu Asn Lys Glu Ala Leu
210 215 220

Glu Leu Leu Lys Thr Ala Ile Ala Lys Ala Gly Tyr Thr Asp Gln Val
225 230 235 240

Val Ile Gly Met Asp Val Ala Ala Ser Glu Phe Tyr Arg Ser Gly Lys
245 250 255

Tyr Asp Leu Asp Phe Lys Ser Pro Asp Asp Pro Ser Arg Tyr Ile Thr
260 265 270

Pro Asp Gln Leu Ala Asp Leu Tyr Lys Ser Phe Val Gln Asn Tyr Pro
275 280 285

Val Val Ser Ile Glu Asp Pro Phe Asp Gln Asp Asp Trp Gly Ala Trp
290 295 300

Gln Lys Phe Thr Ala Ser Ala Gly Ile Gln Val Val Gly Asp Asp Leu

305 310 315 320

Thr Val Thr Asn Pro Lys Arg Ile Ala Lys Ala Ala Ser Glu Lys Ser
325 330 335

Cys Asn Cys Leu Leu Leu Lys Val Asn Gln Ile Gly Ser Val Thr Glu
340 345 350

Ser Leu Gln Ala Cys Lys Leu Ala Gln Ser Asn Gly Trp Gly Val Met
355 360 365

Val Ser His Arg Ser Gly Glu Thr Glu Asp Thr Phe Ile Ala Asp Leu
370 375 380

Val Val Gly Leu Cys Thr Gly Gln Ile Lys Thr Gly Ala Pro Cys Arg
385 390 395 400

Ser Glu Arg Leu Ala Lys Tyr Asn Gln Ile Leu Arg Ile Glu Glu Glu
405 410 415

Leu Gly Ser Lys Ala Lys Phe Ala Gly Arg Ser Phe Arg Asn Pro Leu
420 425 430

Ala Lys

<210> 3
<211> 1755
<212> DNA
<213> Homo sapiens

<400> 3

acggagatct cgccggcttt acgttcacct cggtgtctgc agcaccctcc gcttcctctc 60

ctaggcgacg agacccagtg gctagaagtt caccatgtct attctcaaga tccatgccag 120

ggagatcttt gactctcgcg ggaatcccac tgttgaggtt gatctcttca cctcaaaagg 180

tctcttcaga gctgctgtgc ccagtggtgc ttcaactggt atctatgagg ccctagagct 240

ccgggacaat gataagactc gctatatggg gaagggtgtc tcaaaggctg ttgagcacat 300

caataaaact attgcgcctg ccctggttag caagaaactg aacgtcacag aacaagagaa 360

gattgacaaa ctgatgatcg agatggatgg aacagaaaat aaatctaagt ttggtgcgaa 420

cgccattctg ggggtgtccc ttgccgtctg caaagctggt gccgttgaga agggggtccc 480

cctgtaccgc cacatcgctg acttggctgg caactctgaa gtcatcctgc cagtcccggc 540

gttcaatgtc atcaatggcg gttctcatgc tggcaacaag ctggccatgc aggagttcat 600

gatcctccca gtcggtgcag caaacttcag ggaagccatg cgcattggag cagaggttta 660

ccacaacctg aagaatgtca tcaaggagaa atatgggaaa gatgccacca atgtgggga 720

tgaaggcggg tttgctccca acatcctgga gaataaagaa ggcctggagc tgctgaagac 780

tgctattggg aaagctggct acactgataa ggtggtcatc ggcatggacg tagcggcctc 840

cgagttcttc aggtctggga agtatgacct ggacttcaag tctcccgatg accccagcag 900

gtacatctcg cctgaccagc tggctgacct gtacaagtcc ttcatcaagg actacccagt 960

ggtgtctatc gaagatccct ttgaccagga tgactgggga gcttggcaga agttcacagc 1020

cagtgcagga atccaggtag tgggggatga tctcacagtg accaacccaa agaggatcgc 1080

caaggccgtg aacgagaagt cctgcaactg cctcctgctc aaagtcaacc agattggctc 1140

cgtgaccgag tctcttcagg cgtgcaagct ggcccaggcc aatggttggg gcgtcatggt 1200

gtctcatcgt tcgggggaga ctgaagatac cttcatcgct gacctggttg tggggctgtg 1260

cactgggcag atcaagactg gtgccccttg ccgatctgag cgcttggcca agtacaacca 1320

gctcctcaga attgaagagg agctgggcag caaggctaag tttgccggca ggaacttcag 1380

aaaccccttg gccaagtaag ctgtgggcag gcaagccttc ggtcacctgt tggctacaca 1440

gacccctccc ctcgtgtcag ctcaggcagc tcgaggcccc cgaccaacac ttgcaggggt 1500

ccctgctagt tagcgcccca ccgccgtgga gttcgtaccg cttccttaga acttctacag 1560

aagccaagct ccctggagcc ctgttggcag ctctagcttt tgcagtcgtg taatgggccc 1620

aagtcattgt ttttctcgcc tcactttcca ccaagtgtct agagtcatgt gagcctcgtg 1680

tcatctccgg ggtggccaca ggctagatcc ccggtggttt tgtgctcaaa ataaaaagcc 1740

tcagtgaccc atgag 1755

<210> 4
<211> 430
<212> PRT
<213> Homo sapiens

<220>
<221> CHAIN
<222> (1) .. (430)

<400> 4

```
Met Ser Phe Thr Thr Arg Ser Thr Phe Ser Thr Asn Tyr Arg Ser Leu
1               5                   10                  15

Gly Ser Val Gln Ala Pro Ser Tyr Gly Ala Arg Pro Val Ser Ser Ala
            20                  25                  30

Ala Ser Val Tyr Ala Gly Ala Gly Gly Ser Gly Ser Arg Ile Ser Val
        35                  40                  45

Ser Arg Ser Thr Ser Phe Arg Gly Gly Met Gly Ser Gly Gly Leu Ala
    50                  55                  60

Thr Gly Ile Ala Gly Gly Leu Ala Gly Met Gly Gly Ile Gln Asn Glu
65                  70                  75                  80

Lys Glu Thr Met Gln Ser Leu Asn Asp Arg Leu Ala Ser Tyr Leu Asp
                85                  90                  95

Arg Val Arg Ser Leu Glu Thr Glu Asn Arg Arg Leu Glu Ser Lys Ile
            100                 105                 110

Arg Glu His Leu Glu Lys Lys Gly Pro Gln Val Arg Asp Trp Ser His
        115                 120                 125

Tyr Phe Lys Ile Ile Glu Asp Leu Arg Ala Gln Ile Phe Ala Asn Thr
    130                 135                 140

Val Asp Asn Ala Arg Ile Val Leu Gln Ile Asp Asn Ala Arg Leu Ala
145                 150                 155                 160

Ala Asp Asp Phe Arg Val Lys Tyr Glu Thr Glu Leu Ala Met Arg Gln
                165                 170                 175

Ser Val Glu Asn Asp Ile His Gly Leu Arg Lys Val Ile Asp Asp Thr
            180                 185                 190

Asn Ile Thr Arg Leu Gln Leu Glu Thr Glu Ile Glu Ala Leu Lys Glu
```

195 200 205

Glu Leu Leu Phe Met Lys Lys Asn His Glu Glu Glu Val Lys Gly Leu
210 215 220

Gln Ala Gln Ile Ala Ser Ser Gly Leu Thr Val Glu Val Asp Ala Pro
225 230 235 240

Lys Ser Gln Asp Leu Ala Lys Ile Met Ala Asp Ile Arg Ala Gln Tyr
245 250 255

Asp Glu Leu Ala Arg Lys Asn Arg Glu Glu Leu Asp Lys Tyr Trp Ser
260 265 270

Gln Gln Ile Glu Glu Ser Thr Thr Val Val Thr Thr Gln Ser Ala Glu
275 280 285

Val Gly Ala Ala Glu Thr Thr Leu Thr Glu Leu Arg Arg Thr Val Gln
290 295 300

Ser Leu Glu Ile Asp Leu Asp Ser Met Arg Asn Leu Lys Ala Ser Leu
305 310 315 320

Glu Asn Ser Leu Arg Glu Val Glu Ala Arg Tyr Ala Leu Gln Met Glu
325 330 335

Gln Leu Asn Gly Ile Leu Leu His Leu Glu Ser Glu Leu Ala Gln Thr
340 345 350

Arg Ala Glu Gly Gln Arg Gln Ala Gln Glu Tyr Glu Ala Leu Leu Asn
355 360 365

Ile Lys Val Lys Leu Glu Ala Glu Ile Ala Thr Tyr Arg Arg Leu Leu
370 375 380

Glu Asp Gly Glu Asp Phe Asn Leu Gly Asp Ala Leu Asp Ser Ser Asn
385 390 395 400

Ser Met Gln Thr Ile Gln Lys Thr Thr Thr Arg Arg Ile Val Asp Gly
405 410 415

Lys Val Val Ser Glu Thr Asn Asp Thr Lys Val Leu Arg His
420 425 430

<210> 5
<211> 1439
<212> DNA
<213> Homo sapiens

<400> 5

gcagcctcga gggccaacaa cacctgctgt ccgtgtccat gcccggttgg ccaccccgtt 60

tctgggggca tgagcttcac cactcgctcc accttctcca ccaactaccg gtccctgggc 120

tctgtccagg cgcccagcta cggcgcccgg ccggtcagca gcgcggccag cgtctatgca 180

ggcgctgggg gctctggttc ccggatctcc gtgtcccgct ccaccagctt caggggcggc 240

atggggtccg gggcctggc caccgggata gccgggggtc tggcaggaat gggaggcatc 300

cagaacgaga aggagaccat gcaaagcctg aacgaccgcc tggcctctta cctggacaga 360

gtgaggagcc tggagaccga gaaccggagg ctggagagca aaatccggga gcacttggag 420

aagaagggac cccaggtcag agactggagc cattacttca agatcatcga ggacctgagg 480

gctcagatct tcgcaaatac tgtggacaat gcccgcatcg ttctgcagat tgacaatgcc 540

cgtcttgctg ctgatgactt tagagtcaag tatgagacag agctggccat gcgccagtct 600

gtggagaacg acatccatgg gctccgcaag gtcattgatg acaccaatat cacacgactg 660

cagctggaga cagagatcga ggctctcaag gaggagctgc tcttcatgaa gaagaaccac 720

gaagaggaag taaaaggcct acaagcccag attgccagct ctgggttgac cgtggaggta 780

gatgcccca aatctcagga cctcgccaag atcatggcag acatccgggc ccaatatgac 840

gagctggctc ggaagaaccg agaggagcta gacaagtact ggtctcagca gattgaggag 900

agcaccacag tggtcaccac acagtctgct gaggttggag ctgctgagac gacgctcaca 960

gagctgagac gtacagtcca gtccttggag atcgacctgg actccatgag aaatctgaag 1020

gccagcttgg agaacagcct gagggaggtg gaggcccgct acgccctaca gatggagcag 1080

ctcaacggga tcctgctgca ccttgagtca gagctggcac agacccgggc agagggacag 1140

cgccaggccc aggagtatga ggccctgctg aacatcaagg tcaagctgga ggctgagatc 1200

gccacctacc gccgcctgct ggaagatggc gaggacttta atcttggtga tgccttggac 1260

agcagcaact ccatgcaaac catccaaaag accaccaccc gccggatagt ggatggcaaa 1320

gtggtgtctg agaccaatga caccaaagtt ctgaggcatt aagccagcag aagcagggta 1380

ccctttgggg agcaggaggc caataaaaag ttcagagttc aaaaaaaaaa aaaaaaaaa 1439